Europäisches Patentamt

European Patent Office

Office européen des brevets

(19)

(11) Publication number : **0 623 675 A1**

(12) # EUROPEAN PATENT APPLICATION

(21) Application number : **93308960.9**

(22) Date of filing : **10.11.93**

(51) Int. Cl.$^5$ : **C12N 15/13, C12P 21/08, A61K 47/48, A61K 49/02**

The applicant has filed a statement in accordance with Rule 28 (4) EPC (issue of a sample only to an expert). Accession number(s) of the deposit(s):ATCC 11188, ATCC 11187.

The applicant has subsequently filed a sequence listing and declared, that it includes no new matter.

(30) Priority : **12.11.92 US 975230**

(43) Date of publication of application : **09.11.94 Bulletin 94/45**

(84) Designated Contracting States : **AT BE CH DE DK ES FR GB GR IE IT LI LU NL PT SE**

(71) Applicant : **HYBRITECH INCORPORATED**
**11095 Torreyana Road**
**San Diego, California 92121 (US)**

(72) Inventor : **Ahrweiler, Patricia Marie**
**10335 Devonshire Lane**
**Schaumburg, Illinois 60154 (US)**
Inventor : **Moore, Margaret Dow**
**3616 Jennifer Street**
**San Diego, California 92117 (US)**

(74) Representative : **Hudson, Christopher Mark et al**
**Lilly Industries Limited**
**European Patent Operations**
**Erl Wood Manor**
**Windlesham Surrey GU20 6PH (GB)**
**Declaration under Rule 28(4) EPC (expert solution)**

(54) Altered affinity polypeptides of metal chelate binding antibodies.

(57) This invention provides a polypeptide having an amino acid sequence including a complementarity determining region (CDR) sequence of a native antibody having an amino acid substitution, the polypeptide having a binding affinity for a metal chelate of EDTA or an EDTA analog that is about equal to or altered from the binding affinity of the native antibody.

EP 0 623 675 A1

## BACKGROUND OF THE INVENTION

Throughout this disclosure various publications are referred to by their bibliographic citations within parenthesis. These publications are hereby incorporated by reference to more fully describe the state of the art to which this invention pertains.

The present invention is directed towards antibodies and antibody fragments useful in the diagnosis and treatment of disease. In particular, the present invention is directed towards a family of antibodies or antibody fragments capable of binding metal chelates.

Higher organisms are characterized by an immune system which protects them against invasion by potentially deleterious substances or microorganisms. When a substance, termed an antigen, enters the body, and is recognized as foreign, the immune system mounts both an antibody-mediated response and a cell-mediated response. Cells of the immune system, termed B lymphocytes or B cells, produce antibodies which specifically recognize and bind to the foreign substance. Other lymphocytes, termed T lymphocytes or T cells, both effect and regulate the cell-mediated response resulting eventually in the elimination of the antigen.

It is known that antibody specificity and affinity are governed by the sequence and structure of the complementarity determining regions (CDRs) or hypervariable sequences in the light and heavy variable domains. The structure of the binding region of the antibody involves the variable domains of each pair of light and heavy chains. The domains on the light and heavy chains have the same general structure and each domain comprises four regions having conserved sequences, connected by three hypervariable or complementarity determining regions (CDRs). The four framework regions largely adopt a β-sheet conformation, and the CDRs form loops connecting and in some cases forming part of the β-sheet structure. The CDRs are held in close proximity by the framework regions and, with the CDRs from the other domain, contribute to the formation of the antigen binding site.

Analysis of crystal structures of antibodies complexed with an antigen or hapten have been used to demonstrate that amino acid residues from each CDR contact the antigen (See, for example, Davies et al. J. Biol. Chem. 263 (1988). The interactions between CDR amino acid residues and the hapten molecule have been studied for several antibody-hapten complexes utilizing crystal structures of the complex in conjunction with site-directed mutagenesis studies. (See, for example, Glockshuber et al. Biochemistry 30, 3049-3054 (1991), and Strong et al. Biochemistry 30, 3739-3748 (1991)).

Antibodies are important for both in vitro application in immunoassays and for the in vivo diagnosis and treatment of disease. Antibodies which are directed against a chelate complex of a metal ion and a chelating agent are particularly useful for in vivo imaging and treatment of certain diseases, in particular tumors associated with certain carcinomas such as colorectal and breast carcinomas. Bifunctional antibodies utilizing these metal-specific antibodies also can be constructed that are tumor-specific. Therapeutic or diagnostic radiolabels for localization complexed to appropriate chelating agents can then be delivered in this manner to tumors for imaging or therapeutic purposes.

The present invention fulfills the continuing need for improved therapeutic and diagnostic tools to combat cancer and other diseases.

## SUMMARY OF THE INVENTION

This invention provides a family of polypeptides or variants of an antibody in which the amino acid sequences of the CDRs have been specifically altered such that the binding affinity of the polypeptides for a particular hapten or metal chelate is equal to or altered from the binding affinity of the native antibody.

More particularly, polypeptides or antibody fragments capable of binding EDTA or DOTA metal chelates or related haptens, such that the binding affinity of the polypeptide or antibody fragment is approximately equal to or altered from the native antibody or antibody fragments are provided. The metal complexes include the therapeutic and imaging radionuclides of the metal ion.

In one embodiment, mutant polypeptides of the antibody designated CHA255, a murine monoclonal antibody capable of binding metal complexes are provided. These polypeptides have approximately equivalent, higher or lower binding constants for metal complexes as the native CHA255 antibody. The polypeptides have a particularly high binding affinity for EDTA or DOTA metal complexes, most particularly for the $^{111}$In EDTA or [90]YDOTA complex. It has been found that certain substitutions of amino acids in CDR 1 (H1) and CDR 3 (H3) of the heavy chain, and in CDR 2 (L2) and CDR 3 (L3) of the light chain result in altered affinity polypeptides.

Nucleotide sequences encoding the polypeptides of the present invention, as well as vectors containing these nucleotide sequences are provided. Host cells containing these vectors are further provided.

## BRIEF DESCRIPTION OF THE FIGURES

Fig. 1 shows the amino acid (1A) and nucleic acid (1B) sequences of the light chain variable region of CHA255; and the amino acid (1C) and nucleic acid (1D) sequences of the heavy chain variable region of CHA255.

Fig. 2 shows the amino acid sequences of the light and heavy chain CHA255 CDRs, designated as L1, L2, L3, and H1, H2, and H3.

Fig. 3A shows the positive clones after mutagenesis of L2 and L3, and Fig. 3B shows the positive clones after mutagenesis of H1 and H3.

Fig. 4 shows a comparison of the binding affinities (Ka) of CHA255 native Fab', phage expressed CHA255 native Fab', and phage expressed altered CHA255 Fab' fragments.

## DETAILED DESCRIPTION OF THE INVENTION

Unless defined otherwise, all technical and scientific terms used herein have the same meaning as is commonly understood by one of skill in the art to which this invention pertains.

As used herein, the term "variant," "mutant" or "polypeptides of this invention" refers to a polypeptide or protein having a primary amino acid sequence which is different from that of the native polypeptide or protein. As used herein the term "native" refers to the form of the antibody or fragment thereof that is isolated from nature or that which is without an intentional amino acid substitution.

As used herein, the term "antibody" or "immunoglobulin" refers to a protein that is produced in response to immunization with an antigen or metal chelate, and specifically reacts with the antigen or metal chelate hapten. This includes polyclonal as well as monoclonal antibodies. The antibodies can be isolated from any animal, e.g., a mouse, a rabbit or a human.

As used herein, "antibody" also encompasses fragments of antibodies, also referred to herein as "polypeptides". The antibody fragment or polypeptide retains at least some ability to selectively bind with its antigen or hapten. Also encompassed by this invention are polypeptides that have been recombinantly produced, biochemically synthesized, chemically synthesized or chemically modified, that retain the ability to bind the antigen or hapten of the corresponding native antibody. The ability to bind with an antigen or hapten is determined by antigen-binding assays known in the art such as antibody capture assays (See, for example, Harlow and Lane, Antibodies, A Laboratory Manual, Cold Spring Harbor Laboratory, Cold Spring Harbor, NY (1988)). Antibody fragments or polypeptides retaining some binding affinity include, but are not limited to:

(1) Fab;
(2) Fab';
(3) (Fab')$_2$;
(4) Fv, defined as a genetically engineered fragment containing the variable region of the light chain and the variable region of the heavy chain expressed as two chains; and
(5) SCA, defined as a genetically engineered molecule containing the variable region of the light chain, the variable region of the heavy chain, linked by a suitable polypeptide linker as a genetically fused single chain molecule.

As used herein the terms "light chain variable region" and "heavy chain variable region" refer to the regions or domains at the N-terminal portion of the light and heavy chains respectively which have a varied primary amino acid sequence for each individual native antibody. The variable region of a native antibody consists of the amino terminal domain of the light and heavy chains as they fold together to form a three-dimensional binding site for an antigen.

As used herein the term "monoclonal antibody" refers to immunoglobulins derived from a single clone of cells. All of the monoclonal antibodies derived from the clone are chemically and structurally identical, and specific for a single antigenic determinant.

As used herein the term "hapten" refers to the portion of an antigen that reacts with the immune products of an immune response, but cannot by itself induce an immune response without being complexed to a carrier to form the complete antigen. The metal chelates disclosed by this invention are specific examples of such haptens.

As used herein the term "vector" or "expression vector" refers to sequences of heterologous nucleic acids which are capable of being expressed in selected host cells through operational association with other sequences capable of effecting their expression such as promoter sequences and enhancer sequences. As used herein, the term nucleic acid refers to single or double stranded DNA, cDNA, or RNA. Vectors typically used in recombinant DNA technology include bacterial plasmids, bacterial phages, animal viruses or baculovirus for expression in insect cells.

3

As used herein the term "complementarity determining region" (CDR) or "hypervariable region" refers to amino acid sequences on the light and heavy chains of an antibody which form a three-dimensional loop structure that contributes to the formation of the antigen or hapten binding site.

As used herein the term "CDR grafted" antibody refers to an antibody having an amino acid sequence in which at least parts of one or more CDR sequences in the light and/or variable domain have been replaced by analogous parts of CDR sequences from an antibody having a different binding specificity for a given hapten or antigen. The analogous CDR sequences are said to be "grafted" onto the substrate or recipient antibody (see European Patent Publication No. 0 239 400). The "donor" antibody is the antibody providing the CDR sequence, and the antibody receiving the substituted sequences is the "substrate" antibody.

As used herein the term "chimeric antibody" refers to an antibody in which the variable regions of antibodies derived from one species are combined with the constant regions of antibodies derived from a different species. Chimeric antibodies are constructed by recombinant DNA technology, and are described in Shaw, et al., J. Immun., 138:4534 (1987), Sun, L.K. et al., Proc. Natl. Acad. Sci. USA, 84:214-218 (1987); Neuberger, M.S. et al., Nature, 314:268 (1985), Boulianne, G.L. et al., Nature, 312:643-646 (1984); and Morrison, S.L. et al., Proc. Natl. Acad. Sci. USA, 81:6851-6855 (1984), for example.

As used herein the term "oligonucleotide-directed mutagenesis" or "site-directed mutagenesis" refers to a method of obtaining altered DNA sequences without phenotype selection according to T. Kunkel, Proc. Natl. Acad. Sci. USA 82, pp. 488-492 (1985).

As used herein the term "codon-based mutagenesis" refers to the in vitro substitution of a three nucleotide codon, encoding an amino acid, into an amino acid sequence to produce a mutation in a peptide. This produces an amino acid alteration for each substitution, in contrast to in vivo mutations produced by single nucleotide insertion, substitution, or deletions, which can require a greater number of mutational events in order to produce a change in amino acid sequence.

As used herein the term "metal complex," "metal chelate" or "hapten" refers to chelates of metal ions, for example, chelates of EDTA (ethylenediaminetetraacetic acid) or analogs of EDTA. A number of metals are known to bind EDTA, including In(III), Sc(III), Fe(III), Ga(III), Tb(III), Mn(II), Co(II), Co(III), Cu(II), Zn(II), and Zn(II), as described in U.S. Patent No. 4,722,892. Analogs of EDTA such as diethylenetriaminepentaacetic acid (DTPA), and methods of preparing EDTA and analogs to EDTA are described in U.S. Patent No. 4,622,420. Other analogs to EDTA including DOTA, HETA, TRITA, and TETA are described in U.S. Patent No. 4,678,667. These analogs are capable of complexing with a wide variety of metal ions including radioactive ions as described in U.S. Patent No. 4,622,420. Additional analogs capable of complexing with radioactive metal ions are described in Example 3 below.

As used herein, the term "DOTA" refers to a 2-p-Nitrobenzyl-1,4,7,10-tetraazacyclododecane-N,N',N",N'''-tetraacetic acid. As used herein, the term "EOTUBE" refers to 4-[N'[2-hydroxyethyl]-thioureido]benzyl EDTA, a derivative of p-(aminobenzyl)EDTA which is substituted at one of the internal ethylene carbons. DOTA, when complexed to radioactive yttrium, or EOTUBE, when complexed to radioactive $^{111}$In, is used for various antibody assays as described below. The synthesis of EOTUBE, and its use in a standard Scatchard Analysis, is described infra.

As used herein, the term "association constant", "affinity constant" or "binding constant", Ka, refers to the ratio of the concentration of antibody-metal chelate complex bound over the concentration of free antibody times the concentration of metal chelate complex at equilibrium, as in $\frac{[Ab\cdot H]}{[Ab][H]}$. This is determined, for example, by dialyzing of the antibody and metal chelates to near equilibrium, and quantitating the amount of free antibody or metal chelate (Eisen, Meth. Med. Res. 10, 106 (1964)).

As used herein, the term "metal chelate complex" refers to a complex comprising a polypeptide bound to a metal chelate that is bound to its metal ion.

The polypeptides of the present invention are members of a family of variants of a native antibody or antibody fragment. Each variant contains one or more amino acid substitutions in one or more CDR sequences of the light chain, and/or one or more amino acid substitution or substitutions in one or more CDR sequences of the heavy chain of the native antibody. These polypeptides have a binding affinity for a particular metal chelate which is equivalent to or altered from that of the native antibody.

More particularly, the polypeptides of the present invention are a family of related and therapeutically useful polypeptides or antibody fragments capable of binding metal chelates of EDTA or DOTA or analogs thereof. These polypeptides are useful for in vivo imaging of malignant tissue or tumors. They also are useful for the treatment of malignant tissue or a tumor, for example, colorectal and breast carcinomas. Both methods involve the use of radionuclides which bind to metal chelates or haptens which are specifically delivered to the target site by a targeting molecule. For example, gamma-emitting radionuclides can be complexed with EDTA or EDTA analogs for imaging of targeted tissues. Alpha- or beta- emitting radionuclides can be complexed for radiotherapy of targeted tissues.

In one preferred embodiment, the polypeptides of the present invention are a family of variants of the murine monoclonal antibody CHA255. CHA255 is described in D. T. Reardan et al., Nature 316, pp. 265-268 (1985), and U. S. Patent No. 4,722,892. CHA255 binds to a number of metal ions complexed with EDTA, in particular L-benzyl-EDTA. These include Sc(III), Fe(III), Ga(III), Tb(III), Yb(III), Mn(II), Co(II), Co(III), Cu(II), Zn(II), and Cd(II), (U.S. Patent No. 4,722,892, Table I, column 3). The CHA255 antibody, and the family of CHA255 variants of the present invention have a particularly high binding affinity for the chelate complex of $^{111}$In and L-amino-benzyl EDTA of approximately $10^9 M^{-1}$.

The generation of a family of closely related mutant polypeptides with approximately the same degree of functionality is therapeutically beneficial because it allows for substitution of one antibody for another if the use of a particular antibody causes adverse anti-idiotype reactions in the recipient.

When a native antibody has a low affinity for its hapten, this method is useful to generate derivatives having a higher binding affinity than the native antibody. The increased affinity assures that sufficient quantities of the isotope containing hapten reside at the target for a suitable period of time. Thus, these antibodies then become useful for therapy and imaging.

The generation of a family of mutant polypeptides with lower affinity for the hapten than its corresponding native antibody also can be therapeutically beneficial because there is greater likelihood that the polypeptide will target inside the tumor, thereby improving tumor penetration. Higher affinity polypeptides generally bind strongly to targets on the first available surface, usually the tumor surface, so that the polypeptide does not penetrate well into the tumor.

The polypeptides of the present invention are derived according to the following general strategy. First, nucleic acid sequences encoding the light chain variable region and the heavy chain variable region of a native antibody are cloned into separate vectors. These vectors are subsequently digested and annealed together forming a single expression vector. The nucleic acid sequences within these vectors are mutagenized by in vitro site-directed mutagenesis according to Kunkel et al. supra. In the method of Kunkel et al., a first mutagenized cDNA library contains an array of mutagenized light chain variable regions together with the native heavy chain region to produce a Fab' fragment. Likewise, a second cDNA mutagenized library is produced which contains an array of mutagenized heavy chain variable regions together with the native light variable region to produce a Fab' fragment.

Optionally, of course, a library can be constructed having both light and heavy chain regions mutagenized as described above before selection. Or, alternatively, one can also select the best mutagenized heavy and select the best mutagenized light, combine them, and then select the best combination.

Mutagenesis also is advantageously accomplished by a number of in vitro techniques known in the art such as insertion of random codon cassettes (Lowman et al., Biochemistry 30: 10832-10838 (1991)), or error-prone PCR (Gram et al. Proc. Natl. Acad. Sci. USA 89: 3579-3580.). The entire variable domain can be targeted for mutagenesis, as described, for example, in Jackson et al. Proc. Natl. Acad. Sci. USA 88, pp. 58-62 (1991). Alternatively, if the structure of the native antibody has been sufficiently characterized, certain amino acid sequences of the hypervariable regions can be selectively targeted, for example, as is described in Glockshuber et al. Biochemistry 30: 3049-3054 (1991) for the antibody McPC603. A preferred method for generating and screening a large array of polypeptides produced by mutagenesis is the use of phage display libraries also known as surface display libraries as described in International Publication No. WO 92/06204.

The expression products, assembled as an antibody fragment such as the Fab' fragment, having a potentially functional binding site, are screened for binding affinity by methods known in the art such as ELISAs (Enzyme-Linked Immuno-Sorbent Assay) utilizing the hapten or antigen, or affinity columns (as described, for example, in Skerra and Pluckthun, Science 240, 1038-1041 (1988)). When the polypeptide is derived from native CHA255 antibody, the polypeptides can be screened for affinity hapten binding by a $^{111}$InEOTUBE colony screening assay described below, or by screening with other desired metal hapten complexes. The binding constants of selected clones are determined by Scatchard analysis as described below.

After mutagenesis and selection for binding affinity, nucleic acids encoding the selected polypeptides are isolated: These isolated nucleic acids can be ligated into vectors and introduced into suitable host cells for expression. Methods of ligation and expression of nucleic acids within cells are well known in the art, see Maniatis et al. (1989) (Molecular Cloning: A Laboratory Manual. Cold Spring Harbor Laboratory, Cold Spring Harbor, NY).

Several types of vectors are available and can be used to practice this invention, e.g., plasmid, DNA and RNA viral vectors, baculoviral vectors, and vectors for use in yeast. When the vector is a plasmid, it generally contains a variety of components including promoters, signal sequences, phenotypic selection genes, origin of replication sites, and other necessary components as are known to those of skill in the art.

Promoters most commonly used in prokaryotic vectors include the lac Z promoter system, the alkaline phosphatase pho A promoter, the bacteriophage λPL promoter (a temperature sensitive promoter), the tac promoter (a hybrid trp-lac promoter that is regulated by the lac repressor), the tryptophan promoter, and the bacteriophage T7 promoter.

Preferred promoters used to practice this invention are the lac Z promoter and the pho A promoter. The lac Z promoter is regulated by the lac repressor protein lac i, and thus transcription of the polypeptide can be controlled by manipulation of the level of the lac repressor protein. By way of illustration, a phagemid containing the lac Z promoter is grown in a cell strain that contains a copy of the lac i repressor gene, a repressor for the lac Z promoter. Exemplary cell strains containing the lac i gene include JM 101 and XLI-blue. In the alternative, the host cell can be cotransfected with a plasmid containing both the repressor lac i and the lac Z promoter. Occasionally both of the above techniques are used simultaneously, that is, phagmid particles containing the lac Z promoter are grown in cell strains containing the lac i gene and the cell strains are cotransfected with a plasmid containing both the lac Z and lac i genes. Normally when one wishes to express a gene, to the transfected host above, one would add an inducer such as isopropylthiogalactoside (IPTG), but this step can be omitted.

One other useful component of vectors used to practice this invention is a signal sequence. This sequence is typically located immediately 5' to the gene encoding the polypeptide, and will thus be transcribed at the amino terminus of the fusion protein. However, in certain cases, the signal sequence has been demonstrated to be located at positions other than 5' to the gene encoding the protein to be secreted. This sequence targets the protein to which it is attached across the inner membrane of the bacterial cell. The DNA encoding the signal sequence can be obtained as a restriction endonuclease fragment from any gene encoding a protein that has a signal sequence. Suitable prokaryotic signal sequences can be obtained from genes encoding, for example, LamB or OmpF (Wong et al., Gene 68:193 (1983)), MalE, PhoA, OmpA and other genes. A preferred prokaryotic signal sequence for practicing this invention is the E. coli heat-stable enterotoxin II (STII) signal sequence as described by Chang et al., Gene 55:189 (1987).

Another useful component of the vectors used to practice this invention is a phenotypic selection gene. Typical phenotypic selection genes are those encoding proteins that confer antibiotic resistance upon the host cell. By way of illustration, the ampicillin resistance gene (amp), and the tetracycline resistance gene (tet) are readily employed for this purpose.

Construction of suitable vectors comprising the aforementioned components as well as the gene encoding the desired polypeptide are prepared using standard recombinant DNA procedures. Isolated DNA fragments to be combined to form the vector are cleaved, tailored, and ligated together in a specific order and orientation to generate the desired vector.

The DNA is cleaved using the appropriate restriction enzyme or enzymes in a suitable buffer. In general, about 0.2-1 μg of plasmid or DNA fragments is used with about 1-2 units of the appropriate restriction enzyme in about 20 μl of buffer solution. Appropriate buffers, DNA concentrations, and incubation times and temperatures are specified by the manufacturers of the restriction enzymes. Generally, incubation times of about one or two hours at 37°C are adequate, although several enzymes require higher temperatures. After incubation, the enzymes and other contaminants are removed by extraction of the digestion solution with a mixture of phenol and chloroform and the DNA is recovered from the aqueous fraction by precipitation with ethanol.

To ligate the DNA fragments together to form a functional vector, the ends of the DNA fragments must be compatible with each other. In some cases, the ends will be directly compatible after endonuclease digestion. However, it may be necessary to first convert the sticky ends commonly produced by endonuclease digestion to blunt ends to make them compatible for ligation. To blunt the ends, the DNA is treated in a suitable buffer for at least 15 minutes at 15°C with 10 units of the Klenow fragment of DNA polymerase I (Klenow) in the presence of the four deoxynucleotide triphosphates. The DNA is then purified by phenol-chloroform extraction and ethanol precipitation.

The cleaved DNA fragments are size-separated and selected using DNA gel electrophoresis. The DNA is electrophoresed through either an agarose or a polyacrylamide matrix. The selection of the matrix will depend on the size of the DNA fragments to be separated. After electrophoresis, the DNA is extracted from the matrix by electroelution, or, if low-melting agarose is used as the matrix, by melting the agarose and extracting the DNA from it.

The DNA fragments that are to be ligated together (previously digested with the appropriate restriction enzymes such that the ends of each fragment to be ligated are compatible) are put in solution in about equimolar amounts. The solution will also contain ATP, ligase buffer and a ligase such as T4 DNA ligase at about 10 units per 0.5 μg of DNA. If the DNA fragment is to be ligated into a vector, the vector is at first linearized by cutting with the appropriate restriction endonuclease(s). The linearized vector can then be treated with alkaline phosphatase or calf intestinal phosphatase. The phosphatasing prevents self-ligation of the vector during the ligation step.

After ligation, the vector with the foreign gene now inserted is transformed into a suitable host cell. Suitable prokaryotic host cells include E. coli strain JM101, E. coli K12 strain 294 (ATCC number 31,446), E. coli strain W3110 (ATCC number 27,325), E. coli X1776 (ATCC number 31,537), E. coli XL-1Blue (Stratagene), and E. coli B; however, many other strains of E. coli, such as HB101, NM522, NM538, NM539 and many other species and genera of prokaryotes can be used as well. In addition to the E. coli strains listed above, bacilli such as Bacillus subtillis, other enterobacteriaceae such as Salmonella typhimunium or Serratia marcesans and various Pseudomonas species can all be used as hosts.

Transformation of prokaryotic cells is readily accomplished using calcium chloride or other methods well known to those skilled in the art. Electroporation (Neumann et al., ENBO J. 1:841 (1982)) also can be used to transform these cells. The transformed cells are selected by growth on an antibiotic, commonly tetracycline (tet) or ampicillin (amp), to which they are rendered resistant due to the presence of tet and/or amp resistance genes on the vector.

After selection of the transformed cells, these cells are grown in culture and the plasmid DNA (or other vector with the foreign gene inserted) is then isolated. Plasmid DNA can be isolated using methods known in the art. This purified plasmid DNA is then analyzed by restriction mapping and/or DNA sequencing.

Following procedures outlined above, mammalian cell lines such as myeloma (P3-653), hybridoma (SP2/0), Chinese Hamster Ovary (CHO), Green monkey kidney (COS1) and murine fibroblasts (L492) are suitable host cells for polypeptide expression. These "mammalian" vectors can include a promoter, an enhancer, a polyadenylation signal, signal sequences and genes encoding selectable markers such as geneticin (neomycin resistance), mycophenolic acid (xanthine guanine phosphoribosyl transferase) or histidinol (histidinol dehydrogenase).

Suitable promoters for use in mammalian host cells include, but are not limited to, Ig Kappa, Ig Gamma, Cytomegalovirus (CMV) immediate early, Rous Sarcoma Virus (RSV), Simian virus 40 (SV40) early, mouse mammary tumor (MMTV) virus and metallothionein. Suitable enhancers include, but are not limited to Ig Kappa, Ig Heavy, CMV early and SV40. Suitable polyadenylation sequences include Ig Kappa, Ig Gamma or SV40 large T antigen. Suitable signal sequences include Ig Kappa, Ig Heavy and human growth hormone (HGH).

When the vector is baculovirus, suitable promoters and enhancer sequences include, but are not limited to AcMNPV polyhedrin, AcMNPV ETL and AcMNPV pIO sequences. One particularly suitable polyadenylation signal is the polyhedrin AcMNPV. Ig Kappa, Ig Heavy and AcMNPV are examples of suitable signal sequences. These vectors are useful in the following insect cell lines, among others: SF9, SF21 and High 5.

Alternatively, the polypeptides can be expressed in yeast strains such as PS23-6A, W301-18A, LL20, D234-3, INVSC1, INVSC2, YJJ337. Promoter and enhancer sequences such as gal 1 and pEFT-1 are useful. Vra-4 also provides a suitable enhancer sequence. Sequences useful as functional "origins of replication" include arsl and $2\mu$ circular plasmid.

The preferred murine CHA255 derived polypeptides, having a binding affinity for metal complexes, are derived according to the preferred strategy discussed below. Specific protocols for deriving these mutant polypeptides are presented in Example 1.

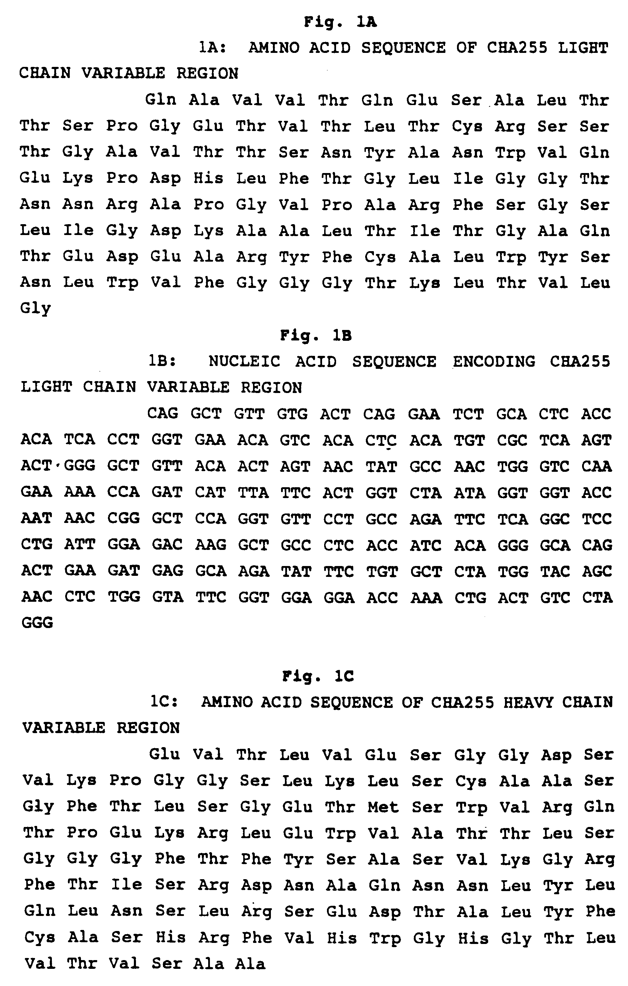

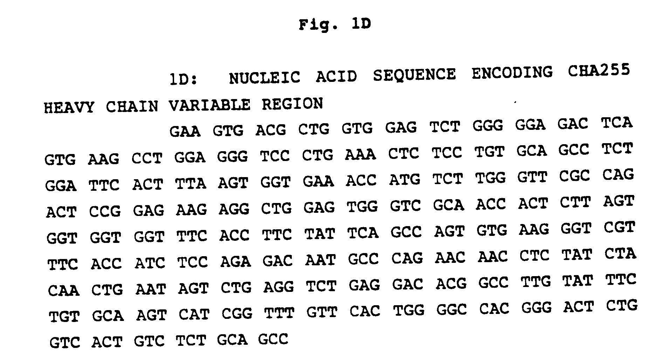

PolyA+ mRNA is isolated from CHA255-expressing hybridoma cells. cDNA synthesis and PCR amplification of the heavy and light chain variable regions are performed as described below. The light and heavy chain variable regions are cloned into appropriate vectors as described below. Sequencing of the cloned CHA variable light and heavy chain genes is accomplished by standard procedures for single stranded templates by the method of Sanger et al., Maniatis, supra. From the cDNA sequence data obtained, the amino acid sequences of the polypeptides encoded by the DNA sequences are deduced by a computer software program, MAPSEQ, commercially available from DNAStar (Madison, Wisconsin.) These sequences are presented in Fig. 1.

The primary amino acid sequence of the light chain variable region of the CHA255 antibody is provided in sequence I.D. No. . The nucleotide sequence encoding the light chain variable region of CHA255 is provided in sequence I.D. No. . The primary amino acid sequence of the heavy chain variable region of the CHA255 antibody is provided in sequence I.D. No. . The nucleotide sequence encoding the heavy chain variable region is provided in sequence I.D. No.

The amino acid sequence of each of the three hypervariable or complementarity-determining regions of both the light and heavy chain variable region is determined. Means to determine the location of the CDR regions are well known to those of skill in the art. For example, CDR locations can be deduced according to the method of Wu and Kabat, J. Exp. Med. 132: 211-250 (1970)).

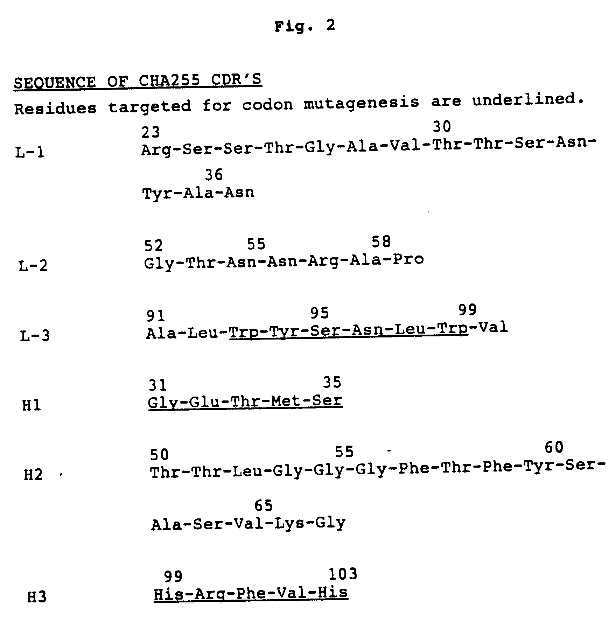

The sequences of the hypervariable regions are provided in Figure 2. L1 is listed as Sequence I.D. No. , L2 is listed as Sequence I.D. No. , L3 is listed as Sequence I.D. No. , H1 is listed as Sequence I.D. No. H2 is listed as Sequence I.D. No. , and H3 is listed as Sequence I.D. No. . Certain amino acid residues of the CDRs thought to be most directly involved in metal chelate binding are targeted for mutagenesis. The residues of the L2 and L3 regions, and the H1 and H3 regions, for example, as underlined in Figure 2, are targeted for mutagenesis.

The preferred method of mutagenizing and screening metal complex binding polypeptides is the cloning of the heavy and light chain variable regions in Fab'-M13 phage display libraries according to the method disclosed in International Application Publication No. WO 92/06204. More specifically, the light chain variable region is ligated into vector IX33 (IXSYS, San Diego, CA), and the heavy chain variable region is ligated into vector IX12 (IXSYS, San Diego, CA). These vectors are then combined into one expression vector by restriction endonuclease digestion, exonuclease digestion, and annealing. This cloning procedure provides a means of selectively mutagenizing the light chain variable region CDRs while maintaining a native heavy chain variable region, and selectively mutagenizing the heavy chain variable region while maintaining the native sequence of the light chain variable region, and allowing an intact Fab' fragment to be expressed on the phage coat for screening.

Mutagenesis of the cloned light and heavy chain variable cDNA can be accomplished by site-directed mutagenesis according to Kunkel et al, supra. The preferred type of mutagenesis is random-codon substitution at a 50% saturation level as described in International Application Publication No. WO 92/06176. This approach has the effect of generating a large library of CDR mutations in CHA255 that is then subjected to screening using replicate filter lift assays to identify mutants with the same or greater affinity as the native.

Specific oligonucleotides synthesized for each of the targeted CDR sequences are given in Example 1 below. International Application Publication No. WO 92/06176 describes the procedure for synthesizing these randomized codons.

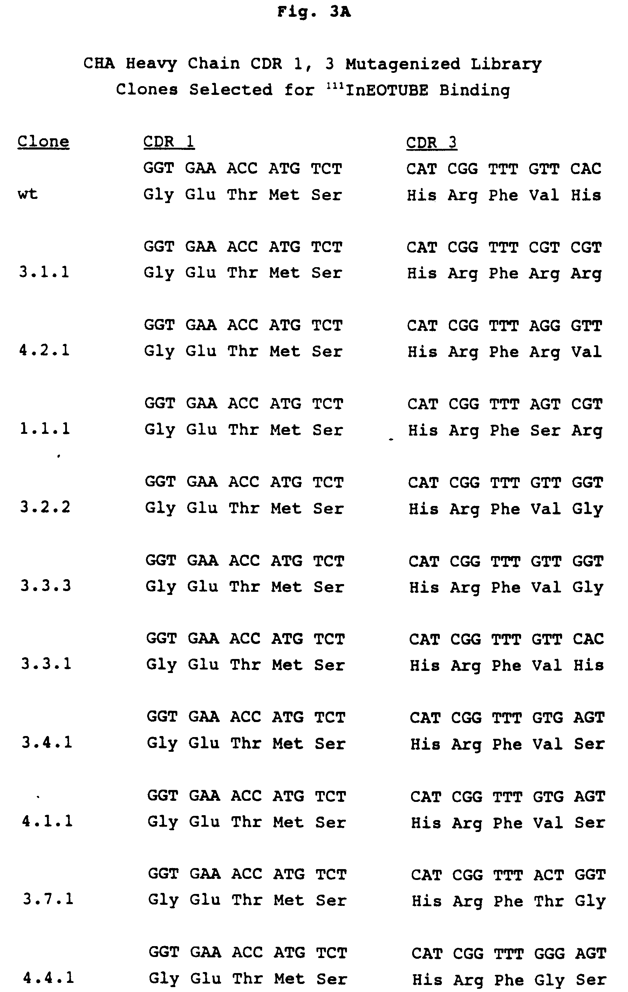

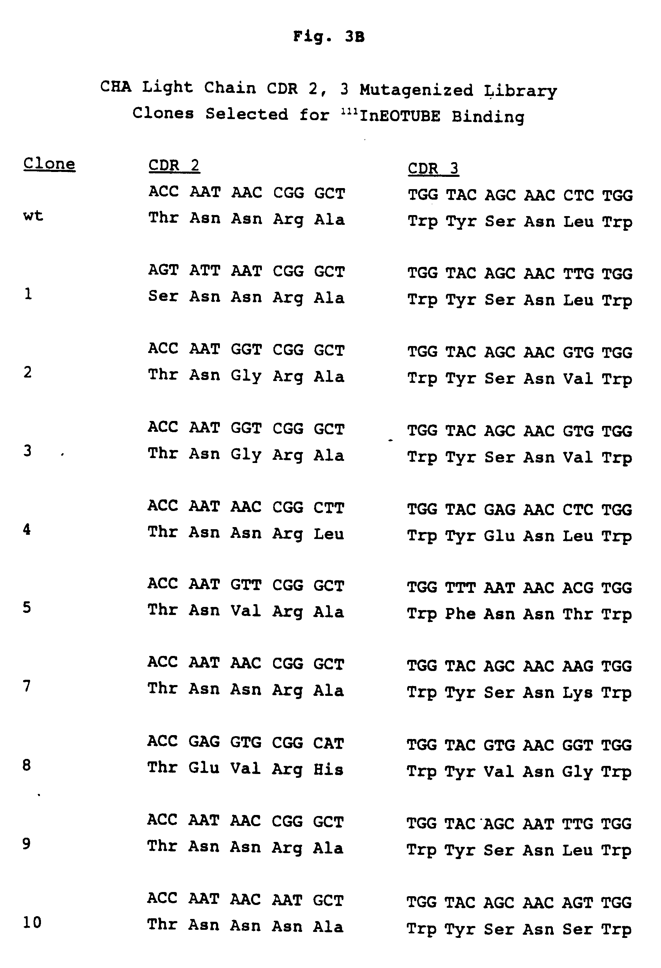

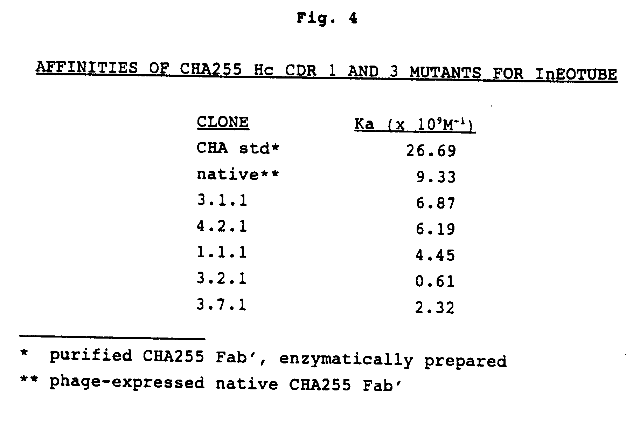

The mutagenized Fab' fragments are expressed and screened with plaque lift assays of nitrocellulose filters, as described in International Application Publication No. WO 92/06204, and identified according to the colony screening assay described in Example 1 below. The amino acid sequences of positive clones identified in $^{111}$InEOTUBE screening assays are determined and are shown in Fig. 3A (light chain CDR 2 and 3) and Fig. 3B (heavy chain CDR 1 and 3). Of the positive clones, the affinity constants of clones 3.1.1, 4.2.1, 1.1.1, 3.2.1, 3.7.1 for InEOTUBE from Fig. 3B are determined using the Scatchard analysis procedure described in Example 2. Fig. 4 shows the results of the Scatchard analysis comparing native CHA255 Fab' fragment, (synthesized, and phage expressed). It can be seen that the binding affinity of each clone analyzed is approximately equivalent, except for clone 3.2.1, which is approximately one order of magnitude less than the native phage expressed Fab'.

Looking at Fig. 4, it can be noted that of the selected clones chosen for the Scatchard analysis, no amino acid substitutions can be observed in H1 or the first three of five H3 amino acids. The last two amino acid residues of H3 are substituted with only three types of residues: arginine (positively charged), serine or threonine (small polar), or glycine or valine (small neutral). However, it is expected that the specific amino acid changes in the CDRs after mutagenesis which result in high binding affinity polypeptides will vary with the native antibody which is used initially.

The polypeptides of the invention can be used to bind a suitable hapten containing an imageable agent. Suitable imageable agents are well known to those of skill in the art and include, but are not limited to, metal ions. Preferred radioactive metal ions for CHA255 derived polypeptides are those which complex with EDTA or EDTA analogs such as $^{111}$In, which emits gamma radiation and is therefore especially useful in radioimaging. When joined to an antibody, antibody fragment, or polypeptide specific to a tumor-associated antigen, the resulting molecule ("bifunctional antibody") acts as a specific delivery system to deliver the imageable agent to the targeted location. Targeted complexes are imaged utilizing Planar Radioimaging, Positron Emission Tomography (PET), NMR for magnetic resonance imaging or Single Photon Emission Computed Tomography (SPECT).

In addition, the polypeptides can bind a suitable hapten containing a therapeutic agent. One preferred group of polypeptides are those which bind DOTA or DOTA analogs containing isotopes which emit alpha- or beta- particles. When the polypeptide is joined to a second antibody specific for a tumor-associated antigen, the molecule ("bifunctional antibody") acts as a tissue-specific delivery system to deliver the therapeutic agent to the targeted location.

More particularly, the polypeptides of the present invention have a binding affinity for metal chelates. The preferred polypeptides are, therefore, those having a specificity for chelating agents that readily bind radioisotopes or other metal ions having some diagnostic or therapeutic usefulness. These agents include chelating agents that can chelate with the radioactive metal ions of $^{195m}$Pt, $^{57}$Ni, $^{57}$Co, $^{105}$Ag, $^{67}$Mn, $^{52}$Fe, $^{111}$In, $^{113m}$In, $^{99m}$Tc, $^{68}$Ga, $^{67}$Ga , $^{169}$Yb, $^{57}$Co, $^{167}$Tm, $^{166}$Tm, $^{146}$Gd, $^{157}$Dy , $^{95m}$Nb, $^{103}$Ru, $^{97}$Ru, $^{99}$Rh, $^{101m}$Rh, $^{201}$Tl, and $^{90}$Y . Most preferred polypeptides are those having an affinity for chelating agents which complex with indium-111 ($^{111}$In), technetium-99m ($^{99m}$Tc), copper-67 ($^{67}$Cu), gallium-67 ($^{67}$Ga), and yttrium-90 ($^{90}$Y). The radioactive metal ion $^{111}$indium(III) and other gamma emitters are generally preferred for diagnostic applications, while $^{90}$yttrium(III) and other beta or alpha emitters are generally preferred for therapeutic applications. For example, EDTA and benzyl EDTA derivatives readily complex $^{111}$In (III) for the desired diagnostic application. Benzyl EDTA, analogs thereof, and methods for their preparation are described in U.S. Patent No. 4,622,420.

In addition, the polypeptides of the present invention can be provided as an intact antibody, or antibody fragment. The antibody or antibody fragment can be constructed from combinations with the native antibody, or combinations with other antibodies having different specificities. For example, the polypeptides of this invention can be incorporated as CDR grafted antibodies, as described below.

Additionally, the polypeptides of this invention are used for the construction of chimeric antibodies. Preferably the CHA255 mutated variable light chain (including mutagenized regions in L1 or L2 or L3) is combined with the constant regions of a human antibody; or the CHA255 mutated variable heavy chain (including mutagenized regions in H1 or H2 or H3) is combined with the constant regions of a human antibody. A hybridoma cell line designated XCHA-351.23 which produces a chimeric antiindium antibody has been deposited with the American Type Culture Collection (ATCC) on November 11 1992, and, accorded ATCC Accession No. 11188. Such chimeric antibodies can be made bifunctional, as described below.

As noted above, the polypeptides of the present invention can be utilized to construct bifunctional antibodies or antibody fragments having dual specificities. The second specificity of a bifunctional antibody will typically be to an antigen associated with a population of malignant cells, a tumor, an infectious organism, or other disease states. See U.S. Patent No. 4,678,667. Examples of tumor-associated antigens are carcinoembryonic antigen (CEA), prostatic acid phosphatase (PAP), prostate specific antigen (PSA), ferritin, bombesin, melanoma associated antigens p97 and gp40, and the like. This "second" specificity is provided by a moiety linked to the polypeptide of this invention. Methods of constructing bifunctional antibodies are generally known in the art, for example, U.S. Patent No. 4,678,667; Parham, J. Immunology, 131, 2895 (1983); Lamoyi et al., J. Immunological Methods, 56, 235 (1983); Parham, id., 53, 133 (1982); and Matthew et al., id., 50 239 (1982). Antibody half molecules also can be used to provide the "second" specificity. See, for example, U. S. Patent No. 4,479,895, and U. S. Patent No. 4,444,878, issued April 24, 1984. Bifunctional antibodies can also be made recombinantly, as is described, for example, in U.S. Patent No. 4,474,893.

Bifunctional specificities can also be achieved using an antibody to a hapten having more than one chelate-specific portion. For example, haptens having designations DB-I to DB-X and DDB-I to DDB-III are particularly preferred compounds having more than one chelate-specific portion. The methods for preparing DB-I to DB-X and DDB-I to DDB-III are described in detail in European Patent Application Publication No. 0 327. 365, published August 8, 1989. These preferred compounds possess a unique "dumbbell" shape due to the joining of the (chelating) hapten with a second, chelating portion on the derivatized hapten. For example, a DB compound could be complexed at the hapten (EDTA) end with a cold (non-radioactive) indium (III) ion, in order to supply the appropriate hapten for the anti-benzyl EDTA-indium antibodies used in this invention, then subsequently the second chelating moiety (e.g., DTPA, DOTA) on the derivatized hapten could be loaded with a radioimaging (e.g., $^{111}$In (III)) metallic nuclide or a radiotherapeutic (e.g., $^{90}$Y (III)) metallic nuclide. Also, the same hapten could be used as a radioimaging agent to target a tumor and further as a radiotherapeutic dose to the imaged tumor. The bispecific antibody containing the polypeptide of this invention can either be loaded with hapten prior to administration or administered without the hapten to allow prelocalization followed at some interval by administration of the labelled hapten.

The haptens generally selected for use in the invention are comprised of chelate complexes of a metal ion and a chelating agent. The chelating agent of the invention is for the hapten portion of the claimed compounds ethylenediaminetetraacetic acid ("EDTA"). EDTA, methods for preparing analogs and the starting materials for the compounds of the present invention are described in U.S. Patent No. 4,622,420, issued November 11, 1986.

Additionally, the derivatized haptens may also include other chelating agents, such as diethylenetriaminepentaacetic acid ("DTPA"), DOTA, HETA, TRITA, TETA and analogs thereof. The methods for preparing DTPA and its analogs are discussed in the '420 patent. The methods for preparing DOTA, HETA, TRITA, TETA, and corresponding starting materials useful in the present invention are described in detail in U.S. Patent No. 4,678,667, issued July 7, 1987 and in Moi et al., J. Am. Chem. Soc., 110:6266 (1988). The EDTA and DTPA analogs disclosed in U.S. Patent No. 4,622,420 are capable of forming physiologically stable chelates with a variety of metal ions. Likewise, the DOTA, HETA, TRITA, and TETA analogs described in U.S. Patent No. 4,678,667 and Moi et al. are capable of forming physiologically stable chelates with various metal ions.

In addition, the polypeptides of the present invention can act as donors or recipients of CDR sequences for altered antibodies as described in European Publication No. 0 239 400 (1987). The peptides can be grafted, for example, with members of the immunoglobulin superfamily. The members of this superfamily include, but are not limited to T-cell receptor, CD4, and CD8. Preferably, the selected CDR sequences listed in Fig. 3 are donor sequences grafted into an appropriate human antibody for therapeutic purposes as set forth in Winter, supra. Such antibodies also can be made bifunctional as described above.

This invention also provides pharmaceutical compositions containing any of the polypeptides described above, alone or in combination with each other, and a pharmaceutically acceptable carrier. As used herein the term "pharmaceutically acceptable carrier" encompasses any of the standard pharmaceutical carriers, such as a phosphate buffered saline solution, water, and emulsions, such as an oil/water or water/oil emulsion, and various types of wetting agents. These pharmaceutical compositions are useful for diagnostic or therapeutic purposes. The polypeptide is present in the pharmaceutical composition in an effective amount. Methods of determining effective amounts are known to those of skill in the art. These pharmaceutical compositions are administered to a subject, e.g. a human patient, for imaging or therapy in a dosage sufficient to elicit the preferred response. Such dosages are easily determined by those skilled in the art. Administration includes, but is not limited to administration orally, intravenously, or parenterally. Administration can be effected continuously or intermittently, such that the amount of the therapeutic or imaging agent in the patient is effective to inhibit the proliferation of the tumor cells or to localize the tumor, respectively.

Nucleic acid sequences encoding the polypeptides described above also are provided by this invention. These nucleotide sequences are determined according to methods known to those of skill in the art, such as described in Example 1. The nucleotide and amino acid sequences of some embodiments are provided in Figures 3A and 3B, in addition to the Sequence I.D. Nos.

The present invention also includes vectors containing the nucleic acid sequences. The vectors are used to transform an appropriate host cell. Techniques by which such vectors are generated and used to transform appropriate host cells are well known to those in the art, see Maniatis et al. supra.

Appropriate host cells for use in production of the polypeptides of the present invention are procaryotic, such as bacterial cell lines, or eucaryotic, such as yeast or insect, or mammalian cell lines, preferably immortalized mammalian cell lines.

A method of recombinantly producing the polypeptides of this invention also is provided. This method requires growing a host cell, described above, under suitable conditions such that the polypeptide is expressed and isolating the polypeptide so produced, see Maniatis, supra. Polypeptides produced by this method also are encompassed by this invention.

The following examples are intended to illustrate but not limit the invention.

**Example 1**

**Construction of CHA255 Mutant Fab' Fragments**

1. Cloning of CHA255 variable regions into **M13** library vectors

Light and heavy CHA255 native variable regions are first cloned into M13 library vectors as follows. PolyA+ mRNA was isolated from approximately CHA255 producing hybridoma cells using a MicroFast Track mRNA Isolation Kit (Invitrogen Corporation, San Diego, CA). First strand cDNA synthesis and PCR amplification of CHA255 heavy and light chain variable regions were performed according to the manufacturer's specifications using the following primers (restriction sites in parentheses are underlined in the sequence):

```
lambda V_L forward (Ncol):
5'-GCCCAACCAGCCATGGCCCAGGCTGTTGTGACTCAGGA-3' (SEQ ID NO.:);


lambda V_L reverse (Xbal):
5'-CCGCTTAACTCTAGACTAGGAACAGTCAGCACGGGAC-3' (SEQ ID NO.:);


heavy chain V_L forward (Xhol):
5'-GAAGTGACGCTGCTCGAGTCTGGGGGAGACT-3' (SEQ ID NO.:);
```

heavy V<sub>L</sub> reverse (Spe I):

5'-TGTGTGAGT<u>ACTAGT</u>ACAACCACAATCCCTGGG-3' (SEQ ID NO.:).

After PCR amplification, CHA255 heavy and light-chain sequences were ligated into heavy and light chain vectors M13IX12 and M13IX33 (IXSYS, Inc., San Diego, CA.), respectively, then combined on one expression vector by restriction endonuclease digestion, exonuclease digestion and annealing according to the procedure of Huse, W.D. "Combinatorial Antibody Expression Libraries in Filamentous Phage", Antibody Engineering: A Practical Guide, C.A.K. Borrsbaeck, Ed., Wm. Freeman and Company, New York, (1991), pp. 103-120. Briefly, the vector into which the heavy chain is ligated contains an M13 coat protein sequence. Translation of the heavy chain produces a gVIII-variable heavy chain fusion protein. Heavy and light chain vector populations are then randomly combined such that the portion of the vectors containing the light and heavy chain variable sequences are joined into a single circular vector as described in International Publication No. WO 92/06204 to Huse. The combined vector directs the coexpression of the light and heavy chain variable sequences for assembly of the polypeptides on the surface of the M13 phage. Panning of high titer phage populations for hapten-binding activity selects the cDNAs encoding the antibody of interest. (Huse, Antibody Engineering, supra, p. 107-109). In this instance, functional [111]InEOTUBE binding clones of the CHA255 antibody were identified on a nitrocellulose filter lift assay. CHA255 native heavy and light chain variable region sequences from a positive clone were verified on single-stranded template with Sequenase™ Version 2 (United States Biochemical Co., Cleveland, OH) according to the manufacturer's instructions.

2. Preparation of mutagenized libraries

The amino acid sequence of the three CDR regions of the CHA255 light chain and the three CDR regions of the heavy chains are given in FIG 1. CDR 1 and 3 of the heavy chain, and CDR 2 and 3 of the light chain were targeted for mutagenesis based on information obtained from X-ray crystal structure of the CHA255 Fab'-hapten complex. Five residues of both CDR 1 and 3 of the CHA255 heavy chain, five of seven residues of light chain CDR 2, and six of nine light chain CDR 3 residues were specifically targeted for codon-based mutagenesis (see underlined residues in Fig. 2). To facilitate screening of the mutagenized library, the targeted CDR sequences were first replaced with a stop codon and Mlu I (heavy) or Hind III (light) restriction site. This template was then used for codon mutagenesis to eliminate wild-type unmutagenized CHA255 Fab' background in the nitrocellulose filter lift assay. This is because the Fab' particles are produced on the surfaces of phage which are grown in Sup E E. coli suppressor strains. Oligonucleotides used for CHA heavy chain CDR1 and 3, and CDR 2 and CDR 3 of the CHA light chain template preparation are as follows (sequences are complementary to the protein coding sequence):

Heavy chain CDR 1 and 3 (Stop/MluI site underlined)

5'CTGGCGAACCCA<u>ACGCGTTTA</u>ACTTAAAGTGAA3' (CDR 1) (SEQ ID NO.:);

5'CCCGTGGCCCCA<u>ACGCGTTTA</u>ACTTGCACAGAA3' (CDR 3) (SEQ ID NO.:).

Light chain CDR 2 and 3 (Stop/Hind III site underlined)

5'AGGAACACCTGG<u>AAGCTTTTA</u>ACCACCTATTAG3' (CDR 2) (SEQ ID NO.: );

5'TCCACCGAATAC<u>AAGCTTTTA</u>TAGAGCACAGAA3' (CDR 3) (SEQ ID NO.: ).

All oligo-directed mutagenesis reactions were done according to Kunkel, T.A., Proc. Natl. Acad. Sci. U.S.A. 82, 488 (1985). Targeted CDR codons were first removed and a Hind III site and stop codon were replaced by oligonucleotide-directed mutagenesis as described by Kunkel above). Heavy and light chain clones with both CDRs replaced with the stop/restriction site sequences were identified by sequencing single-stranded template using Sequenase™ Version 2 (United States Biochemical Co., Cleveland, OH) according to the manufacturer, and used as templates for codon mutagenesis. Codon mutagenesis is described in detail in International Patent Publication No. WO 92/06204.

Mutagenic oligonucleotides with randomized codons were synthesized by B-cyanoethyl phosphoramidite chemistry as is known in the art on a Milligen Cyclone Plus DNA synthesizer (Milligen, Burlington, MA) using a two-column synthesis approach. The two-column oligonucleotide synthesis is described in International Patent Publication No. WO 92/06176 to Huse. The two-column synthesis method produces a mixture of oligonucleotides coding for randomized amino acids within the targeted region while maintaining a 50% bias towards the parental sequence at any position. Resultant mutagenic library clones were screened for heavy and light chain production and [111]InEOTUBE binding.

### 3. Mutant sequence analysis

DNA sequence determinations of mutagenized CHA255 hypervariable regions were done on single-stranded template with Sequenase™ Version 2 (United States Biochemical, Cleveland, OH) and the Genesis 2000 sequencing reagent kit (DuPont Instruments, Wilmington, DE) according to the manufacturer, and analyzed with the Genesis 2000 Automated Sequencer (DuPont Instruments, Wilmington, DE).

### 4. Screening for CHA255 Fab' expression

Expression of heavy and light chains was confirmed with plaque lift assays of nitrocellulose filters (described, for example in International Patent Publication No. WO 92/06204). Briefly, nitrocellulose filters were wetted with 10mM IPTG and blotted dry. The filters were applied to plates with 50-300 plaques and incubated for four hours at room temperature to induce antibody expression. Filters were carefully lifted off the plates, soaked in blocking solution (29 mg/ml of BSA; 3 μg/ml of streptavidin; 0.1 M NaHCO$_3$ pH 8.6-0.02% NaN$_3$) for two hours, and hybridized with goat anti-murine lambda alkaline-phosphatase conjugate (Fisher Scientific Co., Pittsburg, PA) for light chain detection or anti-decapeptide alkaline-phosphatase conjugate for heavy chain detection.

Functional hapten-binding CHA255 clones were identified with an adaptation of an [111]InEOTUBE colony screening assay, described below in Example 3. Briefly, the labelled chelate was prepared by incubating 10 μl of 0.075 mM EOTUBE (N'-(2-hydroxyethyl)-p-thioureidobenzyl EDTA) with 50 μCi of buffered [111]Indium chloride in a metal-free microcentrifuge tube. Blocked filters were incubated with [111]InEOTUBE (10 μCi/filter) in blocking solution for 30 minutes at room temperature. The filters were washed 3X for five minutes with PBS, then dried and exposed overnight to Kodak X-omat AR autoradiography film at -80° C with an intensifying screen.

### 5. ELISA assay of CHA255 Fab' mutants

Antigen (500 ng of BSA-isothiocyanatebenzyl- EDTA-indium antigen dissolved in 50 μl 10 mM NaPO4 per well, (Phelps et al., J. Immunol. 145, 1200-1204 (1990)) was adsorbed to standard 96-well Fisher Titertek plates overnight at 37°C. The wells were washed 1X H$_2$O, 1X PBS, 0.1% TWEEN and incubated with samples of supernatant of phage-infected cultures or periplasmic preparations, for 1-2 hours at room temperature. CHA255 Fab' light chain was detected with goat anti-mouse lambda alkaline-phosphatase conjugate (Fisher Scientific Co., Pittsburg, PA.) diluted 1/250 in 1X PBS, 3% BSA, 3% goat serum one hour at room temperature. Color reagent was added for detection and after approximately 15 minutes, the color was read on a Biotek EL310 Microplate Autoreader (Biotek Instruments, Winooski, VT) at 409 nm.

### 6. Scatchard Analysis of Polypeptides

Free Fab' was expressed and isolated from the periplasmic space of the non-suppressor strain MK30-3 as previously described (International Patent Publication No. WO 92/06204). The concentration of antibody in periplasm preparations was estimated by comparing the ELISA signal generated by aliquots of periplasm of mutant clones to a standard curve using purified CHA255 Fab'. Data points for the Scatchard plots were prepared by incubating antibody (100 μl, approximately 10 nM) diluted in 1X PBS, 1% BSA as crude periplasm fraction with dilutions of cold InEOTUBE (2.43 nM to 0.082 nM) in 1X PBS, 1% BSA hapten spiked with $^{111}$In-EOTUBE as a tracer for 2 hours overnight. This assay is described in greater detail in Example 3, below. The sample was centrifuged at 6,000 rpm for 10 minutes in an Amicon Centrifree™ micro-concentrator (Amicon, Grace Co., Danvers, MA), 10,000 MW cutoff, to separate free hapten from hapten bound to Fab'. Total and free counts were determined and the slope of a plot of the ratio of bound hapten to free hapten versus the concentration of bound hapten was calculated for affinity constant (Ka).

The frequency of $^{111}$In EOTUBE-binding clones detected in the mutagenized heavy chain CDR1 and CDR3 library was approximately 1 to 2 per 1000 plaques per plate screened. The frequency of the [111]InEOTUBE-binding clones detected in the mutagenized light chain CDR 2 and 3 library resulted in approximately 25 per 2000 plaques screened per plate.

Positive plaques with easily identifiable signals were picked and purified in secondary screens and sequence data was confirmed by sequencing at least two clones from the secondary screens.

## Example 2

### Construction of CYIOO1 Mutant CDR Grafted Fab' Fragments

1. Cloning of CYI001 variable regions into pBluescript vectors

A hybridoma cell line producing CYI001 antibody was deposited on under the provisions of the Budapest Treaty on the International Deposit of Microorganisms for the Purposes of Patent Procedure on November 11 1992, under Accession No. 11187. with the American Type Culture Collection, 12301 Parklawn Drive, Rockville, Maryland U.S.A. 20852. Light and heavy CYI001 native variable regions are first cloned into pBluescript vectors as follows. PolyA$^+$ mRNA is isolated from CYI001 expressing hybridoma cells using a MicroFast Track mRNA Isolation Kit (Invitrogen Corporation, San Diego, CA) according to the manufacturer's specifications. First strand cDNA synthesis and PCR amplification of CYI001 heavy and light chain variable regions are performed as previously described above using the following primers (restriction sites in parentheses are underlined in the sequence):

**heavy chain V$_L$ forward (Hind III):**

5'-GCCCAACCAGAAGCTTGATGTGCATCTTCAGGAGTCGGGACCT-3'  (SEQ ID NO.:    );

**heavy chain V$_L$ reverse (Bam HI):**

5'-CCGCTTAACGGATCCTGGATAGACAGATGGGGGTGTCGTTTTGGC-3' (SEQ ID NO.:    );

**lambda V$_L$ forward (Hind III):**

5'-GAAGTGACGCTGAAGCTTCAGGCTGTTGTGACTCAG-3'(SEQ ID NO.: );

**lamda-A V$_L$ reverse (Bam HI):**

5'-TGTGTGAGTGGATCCGAC( T/C )TTGGTACT( G/A )CCGAA-3'  (SEQ ID NO.:    );

```
lambda-B reverse (BamHI):

                          T                 G
5'-TGTGTGAGTGGATCCCAG(   )TTGGTTCC(   )CCGAA-3'  (SEQ ID NO.:
                          C                 A
                                            ‾
)·
```

In the PCR reaction, equal amounts of both lambda $V_L$ reverse primers are used to assure amplification of the lambda V region of interest.

After PCR amplification, heavy- and light-chain sequences are ligated into pBluescript KS(-) vectors (Stratagene, San Diego, CA). DNA sequence determinations of IM-9 cloned heavy and light chain sequences are done on the plasmids with Sequenase™ Version 2 (United States Biochemical, Cleveland, OH) and the Genesis 2000 sequencing reagent kit (DuPont Instruments, Wilmington, DE) according to the manufacturer's instructions and analyzed with the Genesis 2000 Automated Sequencer (DuPont Instruments, Wilmington, DE).

2. Cloning of Human IM-9 Light Chain Gene ($V_KC_K$) and Heavy Chain Gene Fragment ($V_HC_\gamma I$) into M13 Library Vectors

Light IM-9 native gene and heavy IM-9 native gene fragment ($V_HC_\gamma I$) plus the first seven amino acids of the hinge regions are first cloned into M13 library vectors as follows. PolyA$^+$ mRNA is isolated from IM-9 lymphoblastoid cells using a MicroFast Track mRNA Isolation Kit (Invitrogen Corporation, San Diego, CA) according to the manufacturer's specifications. First strand cDNA synthesis and PCR amplification of IM-9 heavy and light chain gene regions are performed as previously described above using the following primers (restriction sites in parentheses are underlined in the sequence):

```
Kappa forward (Ncol):
5'-GCCCAACCAGCCATGGATGGACATGAGGGTCCCC-3'  (SEQ ID NO.:   );


Kappa reverse (Xbal):
5'-CCGCTTAACTCTAGACTAAGACTCTGGCCTGTT-3'  (SEQ ID NO.:   );


heavy chain forward (Xhol):
5'-GAAGTGACGCTGCTCGAGATGGAGTTGGGACTGAGC-3'  (SEQ ID NO.:
);


heavy chain reverse (Spe I):
5'-TGTGTGAGTACTAGTTTAGTCACAAGATTTGGGCTC-3'  (SEQ ID NO.:
).
```

After PCR amplification, IM-9 heavy and light-chain sequences are ligated into heavy and light chain vectors M13IX12 and M13IX33 (IXSYS, Inc., San Diego, CA.), respectively, then combined on one expression vector by annealing according to the procedure of Huse, W.D. "Combinatorial Antibody Expression Libraries in Filamentous Phage", Antibody Engineering: A Practical Guide, C.A.K. Borrsbaeck, Ed., Wm. Freeman and Company, New York, (1991), pp. 103-120. Briefly, the vector into which the heavy chain is ligated contains an M13 coat protein sequence. Translation of the heavy chain produces a gVIII-variable heavy chain fusion protein.

Heavy and light chain vector populations are then randomly combined such that the portion of the vectors containing the light and heavy chain sequences are joined into a single circular vector as described in International Publication No. WO 92/06204. The combined vector directs the coexpression of the light and heavy chain sequences for assembly of the polypeptides on the surface of the M13 phage. IM-9 native heavy and light chain sequences from a positive clone are verified on single-stranded template with Sequenase™ Version 2 (United States Biochemical Co., Cleveland, OH) according to the manufacturer's instructions.

3. CDR Grafting of CYI001/IM-9 Hybrid Expression Vectors.

Given the inferred protein sequences of IM-9 light and heavy chains and CYI001 light and heavy chains, the complementarity determining regions (CDR's) are determined as specified in Kabat et al., (1987) (Seq. of Proteins of Immunol. Interest, NIH, Bethesda, MD) and Wu and Kabat, J. Exp. Med. 132:211-250 (1970). The CDR's in the IM-9 V regions are replaced with the corresponding regions of the CYI001. Simply replacing the sequence defined CDR's can prove inadequate for retention of specificity and affinity. Analysis of antibody X-ray crystal structures has shown that sequence-defined CDR's do not necessarily correspond to antigen binding sites. Consequently, only portions of sequence-defined CDR's can be replaced and some sequence-defined framework residues can be transferred (Verhoeven, M. et al., Science 239:1534-1536 (1988); Jones et al., Nature 321:522-524 (1986); Riechmamm et al., Nature 332:323-327 (1988); Queen et al., Proc. Natl. Acad. Sci. 86:10029-33 (1989)).

Using the protein sequence obtained from modeling, a nucleic acid sequence is derived using a computer software program, MAPSEQ, commercially available from DNAStar (Madison, WI). This sequence is subdivided into overlapping oligonucleotide sequences which are then synthesized and used as templates for polymerase chain reaction to synthesize hybrid V regions containing IM-9 frameworks and CYI001 CDR regions. The V regions are then inserted into the IM-9 expression vectors using double overlap PCR (U.S. Patent 5,023,171).

4. Preparation of mutagenized libraries

The amino acid sequence of the three CDR regions of the CYI001 light chain and the three CDR regions of the heavy chains are determined as described above. In the absence of crystallographic data, CDR regions are defined by analogy to Kabat-defined sequences as defined above. Each of the three CDR's of each chain are individually as described below. Up to five residues of the CDR as targeted for codon mutagenesis.

To facilitate screening of the mutagenized library, the targeted CDR sequences are first replaced with a stop codon and Mlu I (heavy) or Hind III (light) restriction site. This template is then used for codon mutagenesis to eliminate wild-type unmutagenized CDR grafted CYI001/IM-9 Fab' background in the nitrocellulose filter lift assay. This is because the Fab' particles are produced on the surfaces of phage which are grown in Sup E E. coli suppressor strains. Oligonucleotides used for CYI001/IM-9 heavy chain and light chain CDR template preparation are analogous to those described for CHA above.

All oligo-directed mutagenesis reactions are done according to Kunkel, T.A., Proc. Natl. Acad. Sci. U.S.A. 82, 488 (1985). Targeted CDR codons are first removed and a Hind III site and stop codon are replaced by oligonucleotide-directed mutagenesis as described by Kunkel above). Heavy and light chain clones with both CDRs replaced with the stop/restriction site sequences are identified by sequencing single-stranded template using Sequenase™ Version 2 (United States Biochemical Co., Cleveland, OH) according to the manufacturer's instructions, and used as templates for codon mutagenesis. Codon mutagenesis is described in detail in International Patent Publication No. WO 92/06204.

Mutagenic oligonucleotides with randomized codons were synthesized by B-cyanoethyl phosphoramidite chemistry as is known in the art on a Milligen Cyclone Plus DNA synthesizer (Milligen, Burlington, MA) using a two-column synthesis approach. The two-column oligonucleotide synthesis is described in International Patent Publication No. WO 92/06176 to Huse. The two-column synthesis method produces a mixture of oligonucleotides coding for randomized amino acids within the targeted region while maintaining a 50% bias towards the parental sequence at any position.

Resultant mutagenic library clones are screened for heavy and light chain production and $^{90}$Y-NBDOTA binding.

5. Screening for CDR grafted CYI/IM-9 Fab' expression

Expression of heavy and light chains are confirmed with plaque lift assays of nitrocellulose filters (described, for example in International Patent Publication No. WO 92/06204). Briefly, nitrocellulose filters are wetted with 10mM IPTG and blotted dry. The filters are applied to plates with 50-300 plaques and incubated for four hours at room temperature to induce antibody expression. Filters are carefully lifted off the plates, soaked in block solution for two hours, and hybridized with goat anti-murine lambda alkaline-phosphatase conjugate (Fisher Scientific Co., Pittsburg, PA) for light chain detection or anti-decapeptide alkaline-phosphatase conjugate for heavy chain detection.

Functional hapten-binding CDR grafted CYI001/IM-9 clones are identified with an adaptation of an $^{111}$In-EOTUBE colony screening assay, described below in Example 3. Briefly, the labelled chelate is prepared by incubating 10 μl of 0.5 μM NBDOTA with 50 μCi of $^{90}$Y Chloride in 0.01N HCl in a metal-free centrifuge tube. Blocked filters are incubated with $^{90}$Y NBDOTA (3 μCi/filter) in 5% BLOTTO (described in Sambrook et al.) for 30 minutes at room temperature. The filters are washed 3X for five minutes with PBS, then dried and exposed 2 hours to Kodak X-omat AR autoradiography film at -80° C with an intensifying screen. Positive- plaques with easily identifiable signals were picked and purified in secondary screens and sequence data was confirmed by sequencing at least two clones from the secondary screens.

## 6. Mutant sequence analysis

DNA sequence determinations of mutagenized CYI001/IM-9 CDR grafted hypervariable regions are done on single-stranded template with Sequenase™ Version 2 (United States Biochemical, Cleveland, OH) and the Genesis 2000 sequencing reagent kit (DuPont Instruments, Wilmington, DE) according to the manufacturer's instructions, and analyzed with the Genesis 2000 Automated Sequencer (DuPont instruments, Wilmington, DE).

## 7. ELISA assay of CYI001/IM-9 Fab' mutants

Antigen (500 ng of BSA-isothiocyanobenzyl-DOTA-Yttrium) dissolved in 50 μl 10 mM NaPO4 per well, is adsorbed to standard 96-well Fisher Titertek plates overnight at 37°C. The wells are washed 1X $H_2O$, 1X PBS, 0.1% TWEEN and incubated with samples of supernatant of phage-infected cultures or periplasmic preparations, for 1-2 hours at room temperature. CDR grafted CYI001/IM-9 Fab' light chain is detected with goat anti-mouse lambda alkaline-phosphatase conjugate (Fisher Scientific Co., Pittsburg, PA.) diluted 1/250 in 1X PBS, 3% BSA, 3% goat serum one hour at room temperature. Color reagent is added for detection and after approximately 15 minutes, the color is read on a Biotek EL310 Microplate Autoreader (Biotek Instruments, Winooski, VT) at 409 nm.

## 8. Scatchard Analysis of Polypeptides

Free Fab' is expressed and isolated from the periplasmic space of the non-suppressor strain MK30-3 as previously described (International Patent Publication No. WO 92/06204). The concentration of antibody in periplasm preparations is estimated by comparing the ELISA signal generated by aliquots of periplasm of mutant clones to a standard curve using purified CDR grafted CYI/IM-9 Fab'. Data points for the Scatchard plots are prepared by incubating antibody (100 μl, approximately 10 nM) diluted in 1X PBS, 1% BSA as crude periplasm fraction with dilutions of cold Y Nitrobenzyl DOTA in 1X PBS, 1% BSA hapten spiked with $^{90}$Y 2.43 nM to 4.85 μM NBDOTA as a tracer for 2 hours. This type of assay is described in greater detail in Example 3, below. The sample is centrifuged at 6,000 rpm for 10 minutes in an Amicon Centrifree™ micro-concentrator (Amicon, Grace Co., Danvers, MA), 10,000 MW cutoff, to separate free hapten from hapten bound to Fab'. Total and free counts are determined and the slope of a plot of the ratio of bound hapten to free hapten versus the concentration of bound hapten is calculated for affinity constant (Ka).

## Example 3

## Synthesis of EOTUBE and use of EOTUBE in Assays

The indium (III) complex of EOTUBE is used in the qualitative and quantitative assays described herein. EOTUBE is a derivative of p-(aminobenzyl) EDTA. The synthesis of EOTUBE and its use in a standard Scatchard Analysis are set forth below.

Specifically, EOTUBE is EDTA substituted at one of the internal ethylene carbons (S stereochemistry at that substituted carbon) through the benzylic carbon of an N-(2-hydroxyethyl)-N'-(p-benzyl) thio-urea moiety. The synthesis of EOTUBE was performed substantially excluding metal ions. (For example, all glassware was washed with 6M HCl, and only deionized water was used.)

(S)-p-nitrobenzyl EDTA was prepared and converted to (S)-4-isothio-cyanatobenzyl EDTA (hereinafter abbreviated as ITCBE) as described on U. S. Patent No. 4,622,420, and Meares, C. F., _Anal, Biochem., 142,_ 68-75 (1984). The lyophilized ITCBE was resuspended in 0.3M HCl (Ultrex, J. T. Baker, Phillipsburg, N. J.) to a final concentration of approximately 50mM. This solution was stored at -70°C. Unless indicated otherwise, all reactions were preformed in aqueous solution at 40°C.

2.5 ml of 20 mM ITCBE was added to 1.35 ml of 200 mM ethanolamine and the pH adjusted to 11.0 with 10N NaOH. The volume was adjusted to 5 ml with water and the mixture allowed to react for 15 minutes, at which time it was checked by HPLC analysis. All of the ITCBE was converted to EOTUBE, with a retention time of 3.6 minutes on a HPLC (C18 column, eluted with a linear gradient of aqueous buffer of 50 mM triethylammonium acetate to neat methanol on a Hewlett-Packard 1090 instrument).

The product was purified by anion exchange chromatography on an 11 ml column of DEAE Sephadex A-25, eluted with a 110 ml gradient of 0.1 to 1M ammonium formate, pH 6.0 (The column was monitored at 280 nm.) Fractions containing the product were pooled and lyophilized. The product had an absorbance maximum at 246 nm with an extinction coefficient of 18000.

The structure was purified by carbon 13 NM spectroscopy. (A Variant Instruments Model X-300 Mhz instrument was used. The sample was ran in deuteriated water.) The peak corresponding to the carbon in the isothiocyanate moiety in ITCBE was at 139 ppm, and was replaced by a peak at 182 ppm in EOTUBE. This peak corresponds to the carbon in the thiourea linkage. The aromatic region (128-138 ppm) of the spectrum of ITCBE shows four peaks, while that of EOTUBE shows three. In the aliphatic region, there are 5 peaks in common for ITCBE and EOTUBE, and an additional two peaks at 64 and 49 ppm in the EOTUBE spectrum. The latter peaks correspond to the carbons adjacent to the hydroxyl and thio-urea moieties, respectively.

## 1. Radiolabeling of EOTUBE

$9.9540 \times 10^{-7}$ mmoles of EOTUBE was labeled with 600μCi $^{111}$In. The following were added to a metal-free tube, without introducing any other metal:

a. 63μl 0.0158mM metal-free EOTUBE,

b. 63μl 0.26M metal-free ammonium citrate pH=6.0, and

c. 600μCi $^{111}$In ($1.30 \times 10^{-9}$ mmoles).

The mixture was incubated overnight at room temperature. It was then loaded with enough cold indium to give a 1.05 molar ratio of indium (both $^{111}$In and ground state Indium) over EOTUBE. To this tube, 10.2μl 0.012mM ground state InCl$_3$ ($1.044 \times 10^{-6}$ mmoles) was added. It was incubated 4 hours at room temperature. Thin-Layer Chromatography (TLC) analysis was performed to test incorporation of indium. 0.5μl of the labeled sample was spotted in duplicate lanes 1cm from the bottom of a silica gel plate 10.5 inches long marked off in 0.25 inch strips in lanes.

The sample spots were allowed to dry. The plate was placed in a TLC tank with a 10% ammonium acetate:methanol (1:1) solvent system and developed to the 9cm mark. The plate was removed and allowed to dry. Each lane of the plate was cut into 3 sections:

1) The bottom to the 2cm mark was the origin section;

2) 2cm-3.5cm was the middle section;

3) 3.5cm-top is the tail section.

Each section for both lanes were counted.

The percent at the tail for each lane was equal to the percent indium incorporated into the chelate. With indium incorporation greater than 90%, the EOTUBE was diluted to 4.40pg/μl in PBS pH=7.5 for use in the assay.

## 2. Cold Competitor Preparation

$5.530 \times 10^{-4}$ mmoles EOTUBE was loaded with a molar ratio of 1.02X InCl$_3$ over the EOTUBE. Without the introduction of any contaminating metal, the following was added to a metal-free tube:

a. 70μl 7.9mM metal-free EOTUBE ($5.530 \times 10^{-4}$ mmoles) ;

b. 70μl 0.26M metal-free ammonium citrate pH=6.0; and

c. 55.3μl 10.2mM ground state InCl$_3$ ($5.641 \times 10^{-4}$ mmoles) .

The mixture was incubated 4 hours at room temperature. The mixture was then diluted to a 1 ml concentration of 0.40ng/μl and 0.36ng/μl, with PBS pH=7.5 for use in the antibody sensitivity curve.

## 3. Antibody Sensitivity Curve

The following was added to a 96 well microtiter plate, in triplicate:

a. 25μl EOTUBE-$^{111}$In-ground state in at 4.4pg/μl;

b. 25μl antibody dilution (CHA255 or derivative)- either 20μg/ml, 10, 5, 2.5, 1.25, 0.623, 0.313, 0.156, 0.080, 0.040, 0.020, or 0μg/ml;

c. 50μl RPMI with 10% horse serum; and

d. 20μl sheep anti-mouse IgG coupled to sepharose beads (March, S.C., Parikh, I. and Cuatrecacas, P. Analyt. Biochem. 60,149. (1974) diluted to a concentration so that 20μl will bind 0.5μg Ab.

The plates were incubated overnight on a rotator at room temperature. The contents of wells were aspirated onto glass-fiber filter paper, cut out and counted. Antibody dilution number versus fraction bound was graphed to determine the dilution that equals 90% of maximum bound. This value was used for the scatchard assay.

## 4. Scatchard Analysis

The following were added to a 96 well microtiter plate, in triplicate:

a. 25μl EOTUBE-$^{111}$In-ground state in at 4.40pg/μl;

b. 25μl EOTUBE-ground state in at serial dilutions of the 0.40ng/μl and 0.36ng/μl made above. Eleven dilutions each plus a zero giving 24 points with a range of 10ng/well down to 4.40pg/well plus a zero. Dilute with RPMI containing 10% horse serum;

c. 25μl RPMI containing 10% horse serum;

d. 25μl antibody of interest. (concentration determined by sensitivity assay); and

e. 20μl sheep anti-mouse IgG coupled to sepharose beads (same as used in sensitivity curve assay).

The plate was incubated overnight on a rotator at room temperature. The contents of wells were aspirated onto glass-fiber filter paper, cut out and counted. Molar bound vs. bound/free were graphed. The linear regression of the part of the curve that is a straight line was determined. Ka was the negative of the slope.

## Example 4

### Preparation of Bifunctional Antibody ECHO37.2

Bifunctional antibody ECHO37.2 was prepared by the formation of a tetradoma by the biological fusion of the hybridoma cell line expressing monoclonal antibody CHA255, see European Patent Publication No. 0 327 365, published August 9, 1989, and a hybridoma cell line, designated CEM231.6.7, expressing antibody having specificity for carcinoembryonic antigen (CEA). Hybridoma cell line CEM 231.6.7 was deposited with the American Type Culture Collection under the Budapest Treaty on January 7, 1988 (ATCC Accession No. HB 9620). Thus, the bifunctional antibody produced has specificity for both indium benzyl-EDTA and CEA. The tetradoma which expresses the bifunctional antibody designated as ECHO37.2 was prepared by biological fusion of the hybridoma cell lines CHA255 and CEM231.6.7 as described by Burnett, K.G. et al. in Biotechnology: Applications and Research, pp. 401-409, edited by Cheremisinoff and Ouellette, Technomic Publishing Company, Pennsylvania (1985). ECHO37.2 was purified from culture fluid in a two step process. IgG molecules were first isolated from other components of the culture medium by chromatography on DEAE-cellulose, as described by Parham et al., J. Immunol. Meth., 53:133 (1982), followed by further purification by HPLC on hydroxylapatite (BioRad Laboratories, Richmond, CA). The ECHO37.2 bifunctional antibody was eluted using a gradient from 10 mM to 160 mM sodium phosphate, pH 6.8. Both buffers also contained 10 mM Ca$^{2+}$ to prevent column degradation. The bifunctional antibody ECHO37.2 was identified by the pattern of protein bands generated with electrophoresis in 7.5% acrylamide gels (SDS), and the ability of the antibody to bind both indiumbenzyl-EDTA chelates and CEA.

## 1. Hapten Capacity Assay

To determine the ability of the antibody to bind indiumbenzyl-EDTA, the antibody and the In(III) chelate of (S)-4-p-nitrobenzyl EDTA were combined in PBS (10 mM sodium phosphate, 150 mM NaCl, pH 7.5) containing 1.5 mg/mL normal serum albumin. Antibody concentration was 0.15 mg/mL and the Indium (S)-4-p-nitrobenzyl EDTA concentration was 3 μM. The chelate was radiolabeled at a specific activity of 0.5-3.0 μCi/nmol with $^{111}$Indium. After a 5 minute incubation at room temperature, duplicate 400 μl aliquots of the mixture were transferred to separate Centrifree™ ultrafiltration devices (NMW cutoff = 30,000; available from Amicon, Danvers, MA). These were centrifuged at 1500 g for 10 minutes. Duplicate 50 μl pre-Centrifree samples were counted and averaged. This value was called "TC". Duplicate 50 µl aliquots of the Centrifree filtrates were counted and averaged. This value was called "F". The percent of theoretical capacity was calculated from the following equation:

$$(1 - F/TC) \times M$$

M was assigned a value of 150, 100, 200 or 300 for intact antibody (CHA255), Fab fragment, $F(ab)'_2$ bifunctional antibody and intact bifunctional antibody (ECHO37.2), respectively.

To test the ability of the antibody to bind CEA, an immunoreactivity assay was designed to determine the percentage of the radiolabeled anti-CEA antibody preparation which bound to CEA. This value was determined by a two site immunoassay employing solid phase CEA bound to a polystyrene bead by a second antibody reactive with CEA, essentially as described in U.S. 4,376,110. Antigen positive and negative beads were prepared by incubating CEA beads, obtained from the commercially available TANDEM-R CEA test kit (Hybritech Incorporated, San Diego, CA) in high CEA and zero calibrators. After unbound CEA antigen was washed off the beads, approximately 1 ng of the antibody radiolabeled with $^{125}I$ to a specific activity of 10 µCi/µg was added. The percent immunoreactivity was determined by calculating the percent of counts bound to the bead in the presence of antigen. A non-specific binding control was determined by the percent of counts bound to beads that have no CEA antigen present.

## 2. Antibody CHA255 Binding Assay

As indicated, the monoclonal antibody CHA255 is specific for the In(III)-benzyl EDTA complex. The affinity and characterization have been described previously by Reardon et al. in Nature 316:265 (1985). Binding assays were performed to verify the identity of the haptens designated as DB-I to DB-X and DDB-I to DDB-III. Under the conditions of the assay, neither $^{90}$Yttrium (III) or $^{111}$Indium (III) DTPA, alone, bound to CHA255. For the $^{90}$Yttrium (III) hapten chelate, in particular, CHA255 binding required the presence of the Indiumbenzyl-EDTA moiety as provided in the compounds of the invention. The labeled DB and DDB hapten chelates were combined with antibody CHA255 in PBS (10 mM sodium phosphate, 150 mM sodium chloride, pH 7.4) containing 44 µg/mL of human serum albumin and 0.05% Triton-X-100 detergent. Concentrations of the radiolabeled hapten chelating agent and antibody were 5 nM and 133 nM, respectively. The mixture was vortexed and allowed to stand at room temperature for 15 minutes, after which it was transferred to an Amicon Centrifree™ device, with a NMW limit of 30,000, and centrifuged to separate antibody-bound chelate from free hapten chelate. The species bound to the antibody was retained by the membrane and the free chelate flowed through the membrane into the filtrate. The extent of antibody-hapten chelate binding was then determined.

Although the invention has been described with reference to the presently preferred embodiment, it should be understood that various modifications can be made without departing from the spirit of the invention. Accordingly, the invention is limited only by the claims.

## Claims

1. A polypeptide comprising an amino acid sequence including a complementarity determining region (CDR) sequence of a native antibody having an amino acid substitution, the polypeptide having a binding affinity for a metal chelate of EDTA or an EDTA analog, or DOTA or a DOTA analog, that is about equal to or altered from that of the binding affinity of the native antibody.

2. The polypeptide of claim 1, wherein the metal chelate is a metal hapten complex of L-aminobenzyl EDTA.

3. The polypeptide of claim 2, wherein the hapten is the [111]indium chelate of L- or D- (para) aminobenzyl EDTA.

4. The polypeptide of claim 1, wherein the native antibody is a monoclonal antibody.

5. The polypeptide of claim 1, wherein the native antibody is a murine antibody.

6. The polypeptide of claim 5, wherein the native antibody is designated CHA255.

7. The polypeptide of claim 6 wherein the amino acid substitution is in the heavy chain CDR 1 (H1).

8. The polypeptide of claim 6 wherein the amino acid substitution is in the heavy chain CDR 3 (H3).

9. The polypeptide of claim 6 wherein the amino acid substitution is in the light chain CDR 2 (L2).

10. The polypeptide of claim 6 wherein the amino acid substitution is in the light chain CDR 3 (L3).

11. The polypeptide of claim 1, wherein the binding affinity of the polypeptide is greater than the binding affinity of the native antibody.

12. The polypeptide of claim 1, wherein the binding affinity of the polypeptide is less than the binding affinity of the native antibody.

13. A nucleotide sequence encoding the polypeptide of claim 1.

14. A vector containing the nucleic acid sequence of claim 13.

15. A host cell containing the vector of claim 14.

16. A method of producing the polypeptide of claim 1 comprising growing the host cell of claim 15 under suitable conditions permitting production of the polypeptide and recovering the resulting polypeptide.

17. The polypeptide produced by the method of claim 16.

18. The polypeptide of claim 1 bound to an imageable agent.

19. The polypeptide of claim 1 bound to a therapeutic agent.

20. A pharmaceutical composition which comprises the polypeptide of claims 1, 18, or 19 and a pharmaceutically acceptable carrier.

21. A method of delivering a therapeutic agent to a cell which comprises contacting the cell with the pharmaceutical composition of claim 19.

22. The method of claim 21, wherein the contacting is effected _in vitro_.

23. The method of claim 21, wherein the contacting is effected _in vivo_.

24. A method of imaging a target cell in a subject comprising the steps of administering to the subject an effective amount of the composition of claim 18 and then scanning the subject to image the target cell.

## Fig. 1A

1A: AMINO ACID SEQUENCE OF CHA255 LIGHT CHAIN VARIABLE REGION

Gln Ala Val Val Thr Gln Glu Ser Ala Leu Thr
Thr Ser Pro Gly Glu Thr Val Thr Leu Thr Cys Arg Ser Ser
Thr Gly Ala Val Thr Thr Ser Asn Tyr Ala Asn Trp Val Gln
Glu Lys Pro Asp His Leu Phe Thr Gly Leu Ile Gly Gly Thr
Asn Asn Arg Ala Pro Gly Val Pro Ala Arg Phe Ser Gly Ser
Leu Ile Gly Asp Lys Ala Ala Leu Thr Ile Thr Gly Ala Gln
Thr Glu Asp Glu Ala Arg Tyr Phe Cys Ala Leu Trp Tyr Ser
Asn Leu Trp Val Phe Gly Gly Gly Thr Lys Leu Thr Val Leu
Gly

## Fig. 1B

1B: NUCLEIC ACID SEQUENCE ENCODING CHA255 LIGHT CHAIN VARIABLE REGION

CAG GCT GTT GTG ACT CAG GAA TCT GCA CTC ACC
ACA TCA CCT GGT GAA ACA GTC ACA CTC ACA TGT CGC TCA AGT
ACT·GGG GCT GTT ACA ACT AGT AAC TAT GCC AAC TGG GTC CAA
GAA AAA CCA GAT CAT TTA TTC ACT GGT CTA ATA GGT GGT ACC
AAT AAC CGG GCT CCA GGT GTT CCT GCC AGA TTC TCA GGC TCC
CTG ATT GGA GAC AAG GCT GCC CTC ACC ATC ACA GGG GCA CAG
ACT GAA GAT GAG GCA AGA TAT TTC TGT GCT CTA TGG TAC AGC
AAC CTC TGG GTA TTC GGT GGA GGA ACC AAA CTG ACT GTC CTA
GGG

## Fig. 1C

1C: AMINO ACID SEQUENCE OF CHA255 HEAVY CHAIN VARIABLE REGION

Glu Val Thr Leu Val Glu Ser Gly Gly Asp Ser
Val Lys Pro Gly Gly Ser Leu Lys Leu Ser Cys Ala Ala Ser
Gly Phe Thr Leu Ser Gly Glu Thr Met Ser Trp Val Arg Gln
Thr Pro Glu Lys Arg Leu Glu Trp Val Ala Thr Thr Leu Ser
Gly Gly Gly Phe Thr Phe Tyr Ser Ala Ser Val Lys Gly Arg
Phe Thr Ile Ser Arg Asp Asn Ala Gln Asn Asn Leu Tyr Leu
Gln Leu Asn Ser Leu Arg Ser Glu Asp Thr Ala Leu Tyr Phe
Cys Ala Ser His Arg Phe Val His Trp Gly His Gly Thr Leu
Val Thr Val Ser Ala Ala

## Fig. 1D

1D:  NUCLEIC ACID SEQUENCE ENCODING CHA255
HEAVY CHAIN VARIABLE REGION

```
                GAA GTG ACG CTG GTG GAG TCT GGG GGA GAC TCA
GTG AAG CCT GGA GGG TCC CTG AAA CTC TCC TGT GCA GCC TCT
GGA TTC ACT TTA AGT GGT GAA ACC ATG TCT TGG GTT CGC CAG
ACT CCG GAG AAG AGG CTG GAG TGG GTC GCA ACC ACT CTT AGT
GGT GGT GGT TTC ACC TTC TAT TCA GCC AGT GTG AAG GGT CGT
TTC ACC ATC TCC AGA GAC AAT GCC CAG AAC AAC CTC TAT CTA
CAA CTG AAT AGT CTG AGG TCT GAG GAC ACG GCC TTG TAT TTC
TGT GCA AGT CAT CGG TTT GTT CAC TGG GGC CAC GGG ACT CTG
GTC ACT GTC TCT GCA GCC
```

## Fig. 2

SEQUENCE OF CHA255 CDR'S

Residues targeted for codon mutagenesis are underlined.

```
                23                          30
L-1             Arg-Ser-Ser-Thr-Gly-Ala-Val-Thr-Thr-Ser-Asn-
                        36
                Tyr-Ala-Asn


                52          55              58
L-2             Gly-Thr-Asn-Asn-Arg-Ala-Pro


                91                  95              99
L-3             Ala-Leu-Trp-Tyr-Ser-Asn-Leu-Trp-Val


                31                  35
H1              Gly-Glu-Thr-Met-Ser


                50                      55      ·              60
H2  ·           Thr-Thr-Leu-Gly-Gly-Gly-Phe-Thr-Phe-Tyr-Ser-
                        65
                Ala-Ser-Val-Lys-Gly


                99                  103
H3              His-Arg-Phe-Val-His
```

## Fig. 3A

CHA Heavy Chain CDR 1, 3 Mutagenized Library
Clones Selected for [111]InEOTUBE Binding

| Clone | CDR 1 | CDR 3 |
|---|---|---|
| | GGT GAA ACC ATG TCT | CAT CGG TTT GTT CAC |
| wt | Gly Glu Thr Met Ser | His Arg Phe Val His |
| | GGT GAA ACC ATG TCT | CAT CGG TTT CGT CGT |
| 3.1.1 | Gly Glu Thr Met Ser | His Arg Phe Arg Arg |
| | GGT GAA ACC ATG TCT | CAT CGG TTT AGG GTT |
| 4.2.1 | Gly Glu Thr Met Ser | His Arg Phe Arg Val |
| | GGT GAA ACC ATG TCT | CAT CGG TTT AGT CGT |
| 1.1.1 | Gly Glu Thr Met Ser | His Arg Phe Ser Arg |
| | GGT GAA ACC ATG TCT | CAT CGG TTT GTT GGT |
| 3.2.2 | Gly Glu Thr Met Ser | His Arg Phe Val Gly |
| | GGT GAA ACC ATG TCT | CAT CGG TTT GTT GGT |
| 3.3.3 | Gly Glu Thr Met Ser | His Arg Phe Val Gly |
| | GGT GAA ACC ATG TCT | CAT CGG TTT GTT CAC |
| 3.3.1 | Gly Glu Thr Met Ser | His Arg Phe Val His |
| | GGT GAA ACC ATG TCT | CAT CGG TTT GTG AGT |
| 3.4.1 | Gly Glu Thr Met Ser | His Arg Phe Val Ser |
| | GGT GAA ACC ATG TCT | CAT CGG TTT GTG AGT |
| 4.1.1 | Gly Glu Thr Met Ser | His Arg Phe Val Ser |
| | GGT GAA ACC ATG TCT | CAT CGG TTT ACT GGT |
| 3.7.1 | Gly Glu Thr Met Ser | His Arg Phe Thr Gly |
| | GGT GAA ACC ATG TCT | CAT CGG TTT GGG AGT |
| 4.4.1 | Gly Glu Thr Met Ser | His Arg Phe Gly Ser |

42

## Fig. 3B

CHA Light Chain CDR 2, 3 Mutagenized Library
Clones Selected for [111]InEOTUBE Binding

| Clone | CDR 2 | CDR 3 |
|---|---|---|
| wt | ACC AAT AAC CGG GCT<br>Thr Asn Asn Arg Ala | TGG TAC AGC AAC CTC TGG<br>Trp Tyr Ser Asn Leu Trp |
| 1 | AGT ATT AAT CGG GCT<br>Ser Asn Asn Arg Ala | TGG TAC AGC AAC TTG TGG<br>Trp Tyr Ser Asn Leu Trp |
| 2 | ACC AAT GGT CGG GCT<br>Thr Asn Gly Arg Ala | TGG TAC AGC AAC GTG TGG<br>Trp Tyr Ser Asn Val Trp |
| 3 | ACC AAT GGT CGG GCT<br>Thr Asn Gly Arg Ala | TGG TAC AGC AAC GTG TGG<br>Trp Tyr Ser Asn Val Trp |
| 4 | ACC AAT AAC CGG CTT<br>Thr Asn Asn Arg Leu | TGG TAC GAG AAC CTC TGG<br>Trp Tyr Glu Asn Leu Trp |
| 5 | ACC AAT GTT CGG GCT<br>Thr Asn Val Arg Ala | TGG TTT AAT AAC ACG TGG<br>Trp Phe Asn Asn Thr Trp |
| 7 | ACC AAT AAC CGG GCT<br>Thr Asn Asn Arg Ala | TGG TAC AGC AAC AAG TGG<br>Trp Tyr Ser Asn Lys Trp |
| 8 | ACC GAG GTG CGG CAT<br>Thr Glu Val Arg His | TGG TAC GTG AAC GGT TGG<br>Trp Tyr Val Asn Gly Trp |
| 9 | ACC AAT AAC CGG GCT<br>Thr Asn Asn Arg Ala | TGG TAC AGC AAT TTG TGG<br>Trp Tyr Ser Asn Leu Trp |
| 10 | ACC AAT AAC AAT GCT<br>Thr Asn Asn Asn Ala | TGG TAC AGC AAC AGT TGG<br>Trp Tyr Ser Asn Ser Trp |

## Fig. 4

### AFFINITIES OF CHA255 Hc CDR 1 AND 3 MUTANTS FOR InEOTUBE

| CLONE | Ka (x $10^9 M^{-1}$) |
|-------|--------|
| CHA std* | 26.69 |
| native** | 9.33 |
| 3.1.1 | 6.87 |
| 4.2.1 | 6.19 |
| 1.1.1 | 4.45 |
| 3.2.1 | 0.61 |
| 3.7.1 | 2.32 |

\* purified CHA255 Fab', enzymatically prepared

\*\* phage-expressed native CHA255 Fab'

**European Patent Office**

## PARTIAL EUROPEAN SEARCH REPORT

which under Rule 45 of the European Patent Convention shall be considered, for the purposes of subsequent proceedings, as the European search report

Application Number

EP 93 30 8960

### DOCUMENTS CONSIDERED TO BE RELEVANT

| Category | Citation of document with indication, where appropriate, of relevant passages | Relevant to claim | CLASSIFICATION OF THE APPLICATION (Int.Cl.5) |
|---|---|---|---|
| D,Y | PROCEEDINGS OF THE NATIONAL ACADEMY OF SCIENCES OF THE USA<br>vol. 82, no. 2 , 15 January 1985 , WASHINGTON DC, USA<br>pages 488 - 492<br>T. KUNKEL 'Rapid and efficient site-specific mutagenesis without phenotypic selection.'<br>* the whole document *<br>--- | 1-24 | C12N15/13<br>C12P21/08<br>A61K47/48<br>A61K49/02 |
| Y | EP-A-0 327 365 (HYBRITECH INC.)<br>* page 15, line 47 - page 16, line 10 *<br>* page 20, line 20 - page 25, line 29 *<br>* claims *<br>--- | 1-24 | |
| D,A | WO-A-92 06204 (IXSYS, INC.)<br>* the whole document *<br>--- | 1-24 | |
| A | EP-A-0 369 566 (HYBRITECH INC.)<br>* examples 4,5 *<br>* claims *<br>--- | 1-24 | |
| D,A | WO-A-86 01407 (HYBRITECH INC.)<br>* the whole document *<br>----- | 1-24 | TECHNICAL FIELDS SEARCHED (Int.Cl.5)<br><br>C12N<br>C12P<br>A61K |

### INCOMPLETE SEARCH

The Search Division considers that the present European patent application does not comply with the provisions of the European Patent Convention to such an extent that it is not possible to carry out a meaningful search into the state of the art on the basis of some of the claims

Claims searched completely :

Claims searched incompletely :

Claims not searched :

Reason for the limitation of the search:

see sheet C

| Place of search | Date of completion of the search | Examiner |
|---|---|---|
| THE HAGUE | 11 August 1994 | Nooij, F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

45

EP 93 30 8960    -C-

Remarks : Although claim 24 is directed to a diagnostic method practised on the human/animal body, the search has been carried out and based on the alleged effects of the composition/compound.

Although claim 21 (partially, as far as an in vivo method is concerned) and claim 23 (completely) are directed to a method of treatment of the human/animal body, the search has been carried out and based on the alleged effects of the composition/compound.

**SEQUENCE LISTING**

(1) GENERAL INFORMATION

    (i)    APPLICANT: Hybritech Incorporated
        (B)   STREET: 11095 Torreyana Road
        (C)   CITY: San Diego
        (D)   STATE: California
        (E)   COUNTRY: United States of America
        (F)   ZIP: 92121

    (ii)   TITLE OF INVENTION: Altered Affinity Polypeptides
        of Metal Chelate Binding Antibodies

    (iii) NUMBER OF SEQUENCES: 69

    (iv)  CORRESPONDENCE ADDRESS:
        (A)   ADDRESSEE: C. M. Hudson
        (B)   STREET: Erl Wood Manor
        (C)   CITY: Windlesham
        (D)   STATE: Surrey
        (E)   COUNTRY: United Kingdom
        (F)   ZIP: GU20 6PH

    (v)    COMPUTER READABLE FORM:
        (A)   MEDIUM TYPE: Floppy disk
        (B)   COMPUTER: Macintosh
        (C)   OPERATING SYSTEM: Macintosh 7.0
        (D)   SOFTWARE: Microsoft Word 5.1

    (vi)  CURRENT APPLICATION DATA:
        (A)   APPLICATION NUMBER: 93308960.9

(2)    INFORMATION FOR SEQ ID No. 1
    (i)    SEQUENCE CHARACTERISTICS:
        (A)   LENGTH: 110 amino acids
        (B)   TYPE: amino acid
        (D)   TOPOLOGY: linear
    (ii)  MOLECULAR TYPE: protein
    (xi)  SEQUENCE DESCRIPTION: SEQ ID No. 1

```
Gln Ala Val Val Thr Gln Glu Ser Ala Leu Thr Thr Ser Pro Gly
                5                   10                      15
Glu Thr Val Thr Leu Thr Cys Arg Ser Ser Thr Gly Ala Val Thr
                20                  25                      30
Thr Ser Asn Tyr Ala Asn Trp Val Gln Glu Lys Pro Asp His Leu
                35                  40                      45
Phe Thr Gly Leu Ile Gly Gly Thr Asn Asn Arg Ala Pro Gly Val
                50                  55                      60
Pro Ala Arg Phe Ser Gly Ser Leu Ile Gly Asp Lys Ala Ala Leu
                65                  70                      75
Thr Ile Thr Gly Ala Gln Thr Glu Asp Glu Ala Arg Tyr Phe Cys
                80                  85                      90
Ala Leu Trp Tyr Ser Asn Leu Trp Val Phe Gly Gly Gly Thr Lys
                95                  100                     105
Leu Thr Val Leu Gly
                110
```

(2)   INFORMATION FOR SEQ ID No. 2
        (i)    SEQUENCE CHARACTERISTICS:
                (A)    LENGTH:  330 nucleotides
                (B)    TYPE:  nucleic acid
                (C)    STRANDEDNESS:  double
                (D)    TOPOLOGY:  linear
        (ii)   MOLECULAR TYPE:  nucleic acid
        (xi)   SEQUENCE DESCRIPTION:  SEQ ID No. 2

```
CAG GCT GTT GTG ACT CAG GAA TCT GCA CTC ACC ACA TCA CCT GGT    45
GAA ACA GTC ACA CTC ACA TGT CGC TCA AGT ACT GGG GCT GTT ACA    90
ACT AGT AAC TAT GCC AAC TGG GTC CAA GAA AAA CCA GAT CAT TTA   135
TTC ACT GGT CTA ATA GGT GGT ACC AAT AAC CGG GCT CCA GGT GTT   180
CCT GCC AGA TTC TCA GGC TCC CTG ATT GGA GAC AAG GCT GCC CTC   225
ACC ATC ACA GGG GCA CAG ACT GAA GAT GAG GCA AGA TAT TTC TGT   270
GCT CTA TGG TAC AGC AAC CTC TGG GTA TTC GGT GGA GGA ACC AAA   315
CTG ACT GTC CTA GGG                                           330
```

(2)   INFORMATION FOR SEQ ID No. 3
        (i)    SEQUENCE CHARACTERISTICS:
                (A)    LENGTH:  115 amino acids
                (B)    TYPE:  amino acid
                (D)    TOPOLOGY:  linear
        (ii)   MOLECULAR TYPE:  protein
        (xi)   SEQUENCE DESCRIPTION:  SEQ ID No. 3

```
Glu Val Thr Leu Val Glu Ser Gly Gly Asp Ser Val Lys Pro Gly
              5                  10                  15
Gly Ser Leu Lys Leu Ser Cys Ala Ala Ser Gly Phe Thr Leu Ser
             20                  25                  30
Gly Glu Thr Met Ser Trp Val Arg Gln Thr Pro Glu Lys Arg Leu
             35                  40                  45
Glu Trp Val Ala Thr Thr Leu Ser Gly Gly Gly Phe Thr Phe Tyr
             50                  55                  60
Ser Ala Ser Val Lys Gly Arg Phe Thr Ile Ser Arg Asp Asn Ala
             65                  70                  75
Gln Asn Asn Leu Tyr Leu Gln Leu Asn Ser Leu Arg Ser Glu Asp
             80                  85                  90
Thr Ala Leu Tyr Phe Cys Ala Ser His Arg Phe Val His Trp Gly
             95                 100                 105
His Gly Thr Leu Val Thr Val Ser Ala Ala
            110                 115
```

(2)   INFORMATION FOR SEQ ID No. 4
        (i)    SEQUENCE CHARACTERISTICS:
                (A)    LENGTH:  345 nucelic acids
                (B)    TYPE:  nucleic acid
                (C)    STRANDEDNESS:  double
                (D)    TOPOLOGY:  linear
        (ii)   MOLECULAR TYPE:  nucleic acid
        (xi)   SEQUENCE DESCRIPTION:  SEQ ID No. 4

```
GAA GTG ACG CTG GTG GAG TCT GGG GGA GAC TCA GTG AAG CCT GGA    45
GGG TCC CTG AAA CTC TCC TGT GCA GCC TCT GGA TTC ACT TTA AGT    90
GGT GAA ACC ATG TCT TGG GTT CGC CAG ACT CCG GAG AAG AGG CTG   135
GAG TGG GTC GCA ACC ACT CTT AGT GGT GGT GGT TTC ACC TTC TAT   180
TCA GCC AGT GTG AAG GGT CGT TTC ACC ATC TCC AGA GAC AAT GCC   225
CAG AAC AAC CTC TAT CTA CAA CTG AAT AGT CTG AGG TCT GAG GAC   270
ACG GCC TTG TAT TTC TGT GCA AGT CAT CGG TTT GTT CAC TGG GGC   315
CAC GGG ACT CTG GTC ACT GTC TCT GCA GCC                       345
```

21

(2)    INFORMATION FOR SEQ ID No. 5
       (i)    SEQUENCE CHARACTERISTICS:
              (A)    LENGTH:  14 amino acids
              (B)    TYPE:  amino acid
              (D)    TOPOLOGY:  linear
       (ii)   MOLECULAR TYPE:  peptide
       (xi)   SEQUENCE DESCRIPTION:  SEQ ID No. 5


Arg Ser Ser Thr Gly Ala Val Thr Thr Ser Asn
        5                       10
Tyr Ala Asn


(2)    INFORMATION FOR SEQ ID No. 6
       (i)    SEQUENCE CHARACTERISTICS:
              (A)    LENGTH:  7 amino acids
              (B)    TYPE:  amino acid
              (D)    TOPOLOGY:  linear
       (ii)   MOLECULAR TYPE:  peptide
       (xi)   SEQUENCE DESCRIPTION:  SEQ ID No. 6


Gly Thr Asn Asn Arg Ala Pro
        5


(2)    INFORMATION FOR SEQ ID No. 7
       (i)    SEQUENCE CHARACTERISTICS:
              (A)    LENGTH:  9 amino acids
              (B)    TYPE:  amino acid
              (D)    TOPOLOGY:  linear
       (ii)   MOLECULAR TYPE:  peptide
       (xi)   SEQUENCE DESCRIPTION:  SEQ ID No. 7


Ala Leu Trp Tyr Ser Asn Leu Trp Val
        5


(2)    INFORMATION FOR SEQ ID No. 8
       (i)    SEQUENCE CHARACTERISTICS:
              (A)    LENGTH:  5 amino acids
              (B)    TYPE:  amino acid
              (D)    TOPOLOGY:  linear
       (ii)   MOLECULAR TYPE:  peptide
       (xi)   SEQUENCE DESCRIPTION:  SEQ ID No. 8


Gly Glu Thr Met Ser
        5


(2)    INFORMATION FOR SEQ ID No. 9
       (i)    SEQUENCE CHARACTERISTICS:
              (A)    LENGTH:  16 amino acids
              (B)    TYPE:  amino acid
              (D)    TOPOLOGY:  linear
       (ii)   MOLECULAR TYPE:  peptide
       (xi)   SEQUENCE DESCRIPTION:  SEQ ID No. 9


Thr Thr Leu Gly Gly Gly Phe Thr Phe Tyr Ser
        5                   10
Ala Ser Val Lys Gly
            15

(2)    INFORMATION FOR SEQ ID No. 10
    (i)    SEQUENCE CHARACTERISTICS:
        (A)    LENGTH:  5 amino acids
        (B)    TYPE:  amino acid
        (D)    TOPOLOGY:  linear
    (ii)   MOLECULAR TYPE:  peptide
    (xi)   SEQUENCE DESCRIPTION:  SEQ ID No. 10

His Arg Phe Val His
        5


(2)    INFORMATION FOR SEQ ID No. 11
    (i)    SEQUENCE CHARACTERISTICS:
        (A)    LENGTH:  15 nucleotides
        (B)    TYPE:  nucleic acid
        (C)    STRANDEDNESS:  double
        (D)    TOPOLOGY:  linear
    (ii)   MOLECULAR TYPE:  nucleic acid
    (xi)   SEQUENCE DESCRIPTION:  SEQ ID No. 11

GGT GAA ACC ATG TCT 15
Gly Glu Thr Met Ser
        5


(2)    INFORMATION FOR SEQ ID No. 12
    (i)    SEQUENCE CHARACTERISTICS:
        (A)    LENGTH:  15 nucleotides
        (B)    TYPE:  nucleic acid
        (C)    STRANDEDNESS:  double
        (D)    TOPOLOGY:  linear
    (ii)   MOLECULAR TYPE:  nucleic acid
    (xi)   SEQUENCE DESCRIPTION:  SEQ ID No. 12

CAT CGG TTT GTT CAC 15
His Arg Phe Val His
        5


(2)    INFORMATION FOR SEQ ID No. 13
    (i)    SEQUENCE CHARACTERISTICS:
        (A)    LENGTH:  15 nucleotides
        (B)    TYPE:  nucleic acid
        (C)    STRANDEDNESS:  double
        (D)    TOPOLOGY:  linear
    (ii)   MOLECULAR TYPE:  nucleic acid
    (xi)   SEQUENCE DESCRIPTION:  SEQ ID No. 13

GGT GAA ACC ATG TCT 15
Gly Glu Thr Met Ser
        5

(2)    INFORMATION FOR SEQ ID No. 14
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 14

CAT CGG TTT CGT CGT 15
His Arg Phe Arg Arg
        5


(2)    INFORMATION FOR SEQ ID No. 15
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 15

GGT GAA ACC ATG TCT 15
Gly Glu Thr Met Ser
        5


(2)    INFORMATION FOR SEQ ID No. 16
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 16

CAT CGG TTT AGG GTT 15
His Arg Phe Arg Val
        5


(2)    INFORMATION FOR SEQ ID No. 17
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 17

GGT GAA ACC ATG TCT 15
Gly Glu Thr Met Ser
        5

(2)     INFORMATION FOR SEQ ID No. 18
    (i)     SEQUENCE CHARACTERISTICS:
        (A)     LENGTH:   15 nucleotides
        (B)     TYPE:   nucleic acid
        (C)     STRANDEDNESS:   double
        (D)     TOPOLOGY:   linear
    (ii)   MOLECULAR TYPE:   nucleic acid
    (xi)   SEQUENCE DESCRIPTION:   SEQ ID No. 18

CAT CGG TTT AGT CGT 15
His Arg Phe Ser Arg
       5

(2)     INFORMATION FOR SEQ ID No. 19
    (i)     SEQUENCE CHARACTERISTICS:
        (A)     LENGTH:   15 nucleotides
        (B)     TYPE:   nucleic acid
        (C)     STRANDEDNESS:   double
        (D)     TOPOLOGY:   linear
    (ii)   MOLECULAR TYPE:   nucleic acid
    (xi)   SEQUENCE DESCRIPTION:   SEQ ID No. 19

GGT GAA ACC ATG TCT 15
Gly Glu Thr Met Ser
       5

(2)     INFORMATION FOR SEQ ID No. 20
    (i)     SEQUENCE CHARACTERISTICS:
        (A)     LENGTH:   15 nucleotides
        (B)     TYPE:   nucleic acid
        (C)     STRANDEDNESS:   double
        (D)     TOPOLOGY:   linear
    (ii)   MOLECULAR TYPE:   nucleic acid
    (xi)   SEQUENCE DESCRIPTION:   SEQ ID No. 20

CAT CGG TTT GTT GGT 15
His Arg Phe Val Gly
       5

(2)     INFORMATION FOR SEQ ID No. 21
    (i)     SEQUENCE CHARACTERISTICS:
        (A)     LENGTH:   15 nucleotides
        (B)     TYPE:   nucleic acid
        (C)     STRANDEDNESS:   double
        (D)     TOPOLOGY:   linear
    (ii)   MOLECULAR TYPE:   nucleic acid
    (xi)   SEQUENCE DESCRIPTION:   SEQ ID No. 21

GGT GAA ACC ATG TCT 15
Gly Glu Thr Met Ser
       5

(2)     INFORMATION FOR SEQ ID No. 22
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 22

CAT CGG TTT GTT GGT 15
His Arg Phe Val Gly
        5


(2)     INFORMATION FOR SEQ ID No. 23
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 23

GGT GAA ACC ATG TCT 15
Gly Glu Thr Met Ser
        5


(2)     INFORMATION FOR SEQ ID No. 24
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 24

CAT CGG TTT GTT CAC 15
His Arg Phe Val His
        5


(2)     INFORMATION FOR SEQ ID No. 25
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 25

GGT GAA ACC ATG TCT 15
Gly Glu Thr Met Ser
        5

(2)     INFORMATION FOR SEQ ID No. 26
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 26

CAT CGG TTT GTG AGT 15
His Arg Phe Val Ser
        5


(2)     INFORMATION FOR SEQ ID No. 27
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 27

GGT GAA ACC ATG TCT 15
Gly Glu Thr Met Ser
        5


(2)     INFORMATION FOR SEQ ID No. 28
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 28

CAT CGG TTT GTG AGT 15
His Arg Phe Val Ser
        5


(2)     INFORMATION FOR SEQ ID No. 29
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 29

GGT GAA ACC ATG TCT 15
Gly Glu Thr Met Ser
        5

(2)    INFORMATION FOR SEQ ID No. 30
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 30

CAT CGG TTT ACT GGT 15
His Arg Phe Thr Gly
        5


(2)    INFORMATION FOR SEQ ID No. 31
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 31


GGT GAA ACC ATG TCT 15
Gly Glu Thr Met Ser
        5


(2)    INFORMATION FOR SEQ ID No. 32
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 32

CAT CGG TTT GGG AGT 15
His Arg Phe Gly Ser
        5


(2)    INFORMATION FOR SEQ ID No. 33
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 33

ACC AAT AAC CGG GCT 15
Thr Asn Asn Arg Ala
        5

```
(2)     INFORMATION FOR SEQ ID No. 34
        (i)     SEQUENCE CHARACTERISTICS:
                (A)     LENGTH:  18 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 34

TGG TAC AGC AAC CTC TGG 18
Trp Tyr Ser Asn Leu Trp
        5


(2)     INFORMATION FOR SEQ ID No. 35
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 35

AGT ATT AAT CGG GCT 15
Ser Asn Asn Arg Ala
        5


(2)     INFORMATION FOR SEQ ID No. 36
        (i)     SEQUENCE CHARACTERISTICS:
                (A)     LENGTH:  18 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 36

TGG TAC AGC AAC TTG TGG 18
Trp Tyr Ser Asn Leu Trp
        5


(2)     INFORMATION FOR SEQ ID No. 37
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 37

ACC AAT GGT CGG GCT 15
Thr Asn Gly Arg Ala
        5
```

(2) INFORMATION FOR SEQ ID No. 38
    (i) SEQUENCE CHARACTERISTICS:
        (A) LENGTH: 18 nucleotides
        (B) TYPE: nucleic acid
        (C) STRANDEDNESS: double
        (D) TOPOLOGY: linear
    (ii) MOLECULAR TYPE: nucleic acid
    (xi) SEQUENCE DESCRIPTION: SEQ ID No. 38

TGG TAC AGC AAC GTG TGG 18
Trp Tyr Ser Asn Val Trp
    5

2) INFORMATION FOR SEQ ID No. 39
    (i) SEQUENCE CHARACTERISTICS:
        (A) LENGTH: 15 nucleotides
        (B) TYPE: nucleic acid
        (C) STRANDEDNESS: double
        (D) TOPOLOGY: linear
    (ii) MOLECULAR TYPE: nucleic acid
    (xi) SEQUENCE DESCRIPTION: SEQ ID No. 39

ACC AAT GGT CGG GCT 15
Thr Asn Gly Arg Ala
    5

(2) INFORMATION FOR SEQ ID No. 40
    (i) SEQUENCE CHARACTERISTICS:
        (A) LENGTH: 18 nucleotides
        (B) TYPE: nucleic acid
        (C) STRANDEDNESS: double
        (D) TOPOLOGY: linear
    (ii) MOLECULAR TYPE: nucleic acid
    (xi) SEQUENCE DESCRIPTION: SEQ ID No. 40

TGG TAC AGC AAC GTG TGG 18
Trp Tyr Ser Asn Val Trp
    5

(2) INFORMATION FOR SEQ ID No. 41
    (i) SEQUENCE CHARACTERISTICS:
        (A) LENGTH: 15 nucleotides
        (B) TYPE: nucleic acid
        (C) STRANDEDNESS: double
        (D) TOPOLOGY: linear
    (ii) MOLECULAR TYPE: nucleic acid
    (xi) SEQUENCE DESCRIPTION: SEQ ID No. 41

ACC AAT AAC CGG CTT 15
Thr Asn Asn Arg Leu
    5

(2)    INFORMATION FOR SEQ ID No. 42
       (i)    SEQUENCE CHARACTERISTICS:
              (A)    LENGTH:  18 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 42

TGG TAC GAG AAC CTC TGG 18
Trp Tyr Glu Asn Leu Trp
        5


(2)    INFORMATION FOR SEQ ID No. 43
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 43


ACC AAT GTT CGG GCT 15
Thr Asn Val Arg Ala
        5


(2)    INFORMATION FOR SEQ ID No. 44
       (i)    SEQUENCE CHARACTERISTICS:
              (A)    LENGTH:  18 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 44

TGG TTT AAT AAC ACG TGG 18
Trp Phe Asn Asn Thr Trp
        5


(2)    INFORMATION FOR SEQ ID No. 45
       (i)    SEQUENCE CHARACTERISTICS:
              (A)    LENGTH:  15 nucleotides
              (B)    TYPE:  nucleic acid
              (C)    STRANDEDNESS:  double
              (D)    TOPOLOGY:  linear
       (ii)   MOLECULAR TYPE:  nucleic acid
       (xi)   SEQUENCE DESCRIPTION:  SEQ ID No. 45

ACC AAT AAC CGG GCT 15
Thr Asn Asn Arg Ala
        5

(2)     INFORMATION FOR SEQ ID No. 46
        (i)     SEQUENCE CHARACTERISTICS:
                (A)     LENGTH:  18 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 46

TGG TAC AGC AAC AAG TGG 18
Trp Tyr Ser Asn Lys Trp
        5


(2)     INFORMATION FOR SEQ ID No. 47
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 47

ACC GAG GTG CGG CAT 15
Thr Glu Val Arg His
        5


2)      INFORMATION FOR SEQ ID No. 48
        (i)     SEQUENCE CHARACTERISTICS:
                (A)     LENGTH:  18 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 48

TGG TAC GTG AAC GGT TGG 18
Trp Tyr Val Asn Gly Trp
        5


(2)     INFORMATION FOR SEQ ID No. 49
        (i)     SEQUENCE CHARACTERISTICS:
                (A)     LENGTH:  15 nucleotides
                (B)     TYPE:  nucleic acid
                (C)     STRANDEDNESS:  double
                (D)     TOPOLOGY:  linear
        (ii)    MOLECULAR TYPE:  nucleic acid
        (xi)    SEQUENCE DESCRIPTION:  SEQ ID No. 49

ACC AAT AAC CGG GCT 15
Thr Asn Asn Arg Ala
        5

(2)    INFORMATION FOR SEQ ID No. 50
    (i)    SEQUENCE CHARACTERISTICS:
        (A)    LENGTH:  18 nucleotides
        (B)    TYPE:  nucleic acid
        (C)    STRANDEDNESS:  double
        (D)    TOPOLOGY:  linear
    (ii)   MOLECULAR TYPE:  nucleic acid
    (xi)   SEQUENCE DESCRIPTION:  SEQ ID No. 50

TGG TAC AGC AAT TTG TGG 18
Trp Tyr Ser Asn Leu Trp
      5

(2)    INFORMATION FOR SEQ ID No. 51
    (i)    SEQUENCE CHARACTERISTICS:
        (A)    LENGTH:  15 nucleotides
        (B)    TYPE:  nucleic acid
        (C)    STRANDEDNESS:  double
        (D)    TOPOLOGY:  linear
    (ii)   MOLECULAR TYPE:  nucleic acid
    (xi)   SEQUENCE DESCRIPTION:  SEQ ID No. 51

ACC AAT AAC AAT GCT 15
Thr Asn Asn Asn Ala
      5

(2)    INFORMATION FOR SEQ ID No. 52
    (i)    SEQUENCE CHARACTERISTICS:
        (A)    LENGTH:  18 nucleotides
        (B)    TYPE:  nucleic acid
        (C)    STRANDEDNESS:  double
        (D)    TOPOLOGY:  linear
    (ii)   MOLECULAR TYPE:  nucleic acid
    (xi)   SEQUENCE DESCRIPTION:  SEQ ID No. 52

TGG TAC AGC AAC AGT TGG 18
Trp Tyr Ser Asn Ser Trp
      5

(2)    INFORMATION FOR SEQ ID No. 53
    (i)    SEQUENCE CHARACTERISTICS:
        (A)    LENGTH:  39 nucleotides
        (B)    TYPE:  nucleic acid
        (C)    STRANDEDNESS:  single
        (D)    TOPOLOGY:  linear
    (ii)   MOLECULAR TYPE:  nucleic acid
    (xi)   SEQUENCE DESCRIPTION:  SEQ ID No. 53

GCCCAACCAG CCCATGGCCC AGGCTGTTGT GACTCAGGA 39

```
(2)     INFORMATION FOR SEQ ID No. 54
        (i)     SEQUENCE CHARACTERISTICS:
                (A)     LENGTH:  37 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 54
```

CCGCTTAACT CTAGACTAGG AACAGTCAGC ACGGGAC 37

```
(2)     INFORMATION FOR SEQ ID No. 55
        (i)     SEQUENCE CHARACTERISTICS:
                (A)     LENGTH:  31 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 55
```

GAAGTGACGC TGCTCGAGTC TGGGGGAGAC T 31

```
(2)     INFORMATION FOR SEQ ID No. 56
        (i)     SEQUENCE CHARACTERISTICS:
                (A)     LENGTH:  33 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 56
```

TGTGTGAGTA CTAGTACAAC CACAATCCCT GGG 33

```
(2)     INFORMATION FOR SEQ ID No. 57
        (i)     SEQUENCE CHARACTERISTICS:
                (A)     LENGTH:  33 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 57
```

CTGGCGAACC CAACGCGTTT AACTTAAAGT GAA   33

```
(2)     INFORMATION FOR SEQ ID No. 58
        (i)     SEQUENCE CHARACTERISTICS:
                (A)     LENGTH:  33 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 58
```

CCCGTGGCCC CAACGCGTTT AACTTGCACA GAA   33

34

(2)  INFORMATION FOR SEQ ID No. 59
      (i)   SEQUENCE CHARACTERISTICS:
            (A)   LENGTH:  33 nucleotides
            (B)   TYPE:  nucleic acid
            (C)   STRANDEDNESS:  single
            (D)   TOPOLOGY:  linear
      (ii)  MOLECULAR TYPE:  nucleic acid
      (xi)  SEQUENCE DESCRIPTION:  SEQ ID No. 59

AGGAACACCT GGAAGCTTTT AACCACCTAT TAG   33


(2)  INFORMATION FOR SEQ ID No. 60
      (i)   SEQUENCE CHARACTERISTICS:
            (A)   LENGTH:  33 nucleotides
            (B)   TYPE:  nucleic acid
            (C)   STRANDEDNESS:  single
            (D)   TOPOLOGY:  linear
      (ii)  MOLECULAR TYPE:  nucleic acid
      (xi)  SEQUENCE DESCRIPTION:  SEQ ID No. 60

TCCACCGAAT ACAAGCTTTT ATAGAGCACA GAA   33


(2)  INFORMATION FOR SEQ ID No. 61
      (i)   SEQUENCE CHARACTERISTICS:
            (A)   LENGTH:  43 nucleotides
            (B)   TYPE:  nucleic acid
            (C)   STRANDEDNESS:  single
            (D)   TOPOLOGY:  linear
      (ii)  MOLECULAR TYPE:  nucleic acid
      (xi)  SEQUENCE DESCRIPTION:  SEQ ID No. 61

GCCCAACCAG AAGCTTGATG TGCATCTTCA GGAGTCGGGA CCT 43


(2)  INFORMATION FOR SEQ ID No. 62
      (i)   SEQUENCE CHARACTERISTICS:
            (A)   LENGTH:  45 nucleotides
            (B)   TYPE:  nucleic acid
            (C)   STRANDEDNESS:  single
            (D)   TOPOLOGY:  linear
      (ii)  MOLECULAR TYPE:  nucleic acid
      (xi)  SEQUENCE DESCRIPTION:  SEQ ID No. 62

CCGCTTAACG GATCCTGGAT AGACAGATGG GGGTGTCGTT TTGGC 45


(2)  INFORMATION FOR SEQ ID No. 63
      (i)   SEQUENCE CHARACTERISTICS:
            (A)   LENGTH:  36 nucleotides
            (B)   TYPE:  nucleic acid
            (C)   STRANDEDNESS:  single
            (D)   TOPOLOGY:  linear
      (ii)  MOLECULAR TYPE:  nucleic acid
      (xi)  SEQUENCE DESCRIPTION:  SEQ ID No. 63

GAAGTGACGC TGAAGCTTCA GGCTGTTGTG ACTCAG   36

(2)     INFORMATION FOR SEQ ID No. 64
        (i)     SEQUENCE CHARACTERISTICS:
                (A)     LENGTH:  33 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 64


TGTGTGAGTG GATCCGACBT TGGTACTRCC GAA   33


(2)     INFORMATION FOR SEQ ID No. 65
        (i)     SEQUENCE CHARACTERISTICS:
                (A)     LENGTH:  33 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 65


TGTGTGAGTG GATCCCAGBT TGGTTCCRCC GAA 33


(2)     INFORMATION FOR SEQ ID No. 66
        (i)     SEQUENCE CHARACTERISTICS:
                (A)     LENGTH:  34 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 66


GCCCAACCAG CCATGGATGG ACATGAGGGT CCCC 34


(2)     INFORMATION FOR SEQ ID No. 67
        (i)     SEQUENCE CHARACTERISTICS:
                (A)     LENGTH:  33 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 67


CCGCTTAACT CTAGACTAAG ACTCTGGCCT GTT 33


(2)     INFORMATION FOR SEQ ID No. 68
        (i)     SEQUENCE CHARACTERISTICS:
                (A)     LENGTH:  36 nucleotides
                (B)     TYPE:  nucleic acid
                (C)     STRANDEDNESS:  single
                (D)     TOPOLOGY:  linear
        (ii)    MOLECULAR TYPE:  nucleic acid
        (xi)    SEQUENCE DESCRIPTION:  SEQ ID No. 68


GAAGTGACGC TGCTCGAGAT GGAGTTGGGA CTGAGC 36

```
(2)    INFORMATION FOR SEQ ID No. 69
       (i)    SEQUENCE CHARACTERISTICS:
              (A)   LENGTH:  36 nucleotides
              (B)   TYPE:  nucleic acid
              (C)   STRANDEDNESS:  single
              (D)   TOPOLOGY:  linear
       (ii)   MOLECULAR TYPE:  nucleic acid
       (xi)   SEQUENCE DESCRIPTION:  SEQ ID No. 69

   TGTGTGAGTA CTAGTTTAGT CACAAGATTT GGGCTC 36
```